# EUROPEAN PATENT SPECIFICATION

(11) **EP 1 631 561 B1**
(45) Date of publication and mention of the grant of the patent: **18.08.2010**
(21) Application number: 04750543.3
(22) Date of filing: 23.04.2004
(51) Int. Cl.: C07D 307/80, C07D 307/79, C07D 413/04, C07D 417/04, C07D 405/04, A61K 51/04

(54) **COMPOSITIONS AND METHODS FOR NON-INVASIVE IMAGING OF SOLUBLE BETA-AMYLOID**
ZUSAMMENSETZUNGEN UND VERFAHREN ZUR IN VIVO ABBILDUNG VON LOESLICHEN BETA-AMYLOID
COMPOSITIONS ET PROCEDES DE VISUALISATION NON EFFRACTIVE DU BETA-AMYLOIDE SOLUBLE

(30) Priority: 07.05.2003 US 431202; 26.12.2003 US 747715
(43) Date of publication of application: 08.03.2006
(73) Proprietor: GENERAL ELECTRIC COMPANY, Schenectady, NY 12345 (US)
(72) Inventor: MONTALTO, Michael, Christopher, Colonie, NY 12205 (US); AGDEPPA, Eric, Dustin, Latham, NY 12110 (US); SICLOVAN, Tiberiu, Mircea, Rexford, NY 12148 (US); WILLIAMS, Amy, Casey, Clifton Park, NY 12065 (US)
(74) Representative: Canning, Lewis R.
(86) International application number: PCT/US2004/012559
(87) International publication number: WO 2004/100998

(56) References cited:
- WO-A-02/16333
- WO-A-02/085903
- WO-A-03/051859
- HOWLETT D R ET AL: "INHIBITION OF FIBRIL FORMATION IN BETA-AMYLOID PEPTIDE BY A NOVEL SERIES OF BENZOFURANS" BIOCHEMICAL JOURNAL, PORTLAND PRESS, LONDON, GB, vol. 340, no. 1, 1999, pages 283-289, XP009006358 ISSN: 0264-6021
- DATABASE CROSSFIRE BEILSTEIN INSTITUTE FOR ORGANIC CHEMISTRY, FRANKFURT-MAIN, DE; XP002300651 Database accession no. BRN:169730 & J. AMER. CHEM. SOC., vol. 96, 1974, pages 5495-5508,
- DATABASE CROSSFIRE BEILSTEIN INSTITUTE FOR ORGANIC CHEMISTRY, FRANKFURT-MAIN, DE; XP002300652 Database accession no. BRN:5510834 & J. ORG. CHEM., vol. 51, no. 25, 1986, pages 5040-5041,
- DATABASE CROSSFIRE BEILSTEIN INSTITUTE FOR ORGANIC CHEMISTRY, FRANKFURT-MAIN, DE; XP002300653 Database accession no. BRN: 3551523 & BULL. ACAD. SCI., vol. 38, no. 3.2, 1989, pages 579-580,

## Description

### FIELD OF THE INVENTION

The present disclosure relates to the detection of soluble beta-amyloid and novel compositions, imaging agents and benzofuran derivative relating thereto.

### BACKGROUND OF THE INVENTION

The main histopathological characteristics of Alzheimer's disease ("AD") are the presence of neuritic plaques and tangles combined with associated inflammation in the brain. It is known that plaques are composed mainly of deposited (or insoluble in aqueous solution) fibrillar forms of the beta-amyloid ("A-beta") peptide. The formation of fully fibrillar aggregated A-beta peptide is a complex process that is initiated by the cleavage of the amyloid precursor protein ("APP"). After cleavage of APP, the monomeric form of A-beta can associate with other monomers, presumably through hydrophobic interactions and/or domain swapping, to form dimers, trimers and higher order oligomers. Oligomers of A-beta can further associate to form protofibrils and eventual fibrils, which is the main constituent of neuritic plaques. It has recently been shown that soluble oligomers (soluble in aqueous buffer) of A-beta may contribute significantly to neuronal dysfunction. In fact, animal models suggest that simply lowering the amount of soluble A-beta peptide, without affecting the levels of A-beta in plaques, may be sufficient to improve cognitive function. Presently, the only definitive method of AD diagnosis is postmortem examination of brain for the presence of plaques and tangles. The antemortem diagnosis of AD is difficult, especially during the early stages, as AD symptoms are shared among a spectrum of other dementias. Currently, AD diagnosis is achieved using simple cognitive tests designed to test a patient's mental capacity such as, for example, the ADAS-cog (Alzheimer's disease assessment scale - cognitive subscale) or MMSE (Mini-mental state examination). The subjective nature and inherent patient variability is a major shortcoming of diagnosing AD by such means. The fact that AD cannot be accurately diagnosed early creates a formidable challenge for pharmaceutical companies that aim to test anti-A-beta drugs as therapy to slow or halt AD pathogenesis. Furthermore, even if AD could be detected early and patients could be treated with A-beta lowering compounds, there is currently no way to know if the therapy is clinically efficacious. Therefore, a significant need exists to develop methods of measuring the soluble A-beta peptide levels locally in the brain.

Diagnosing AD by directly measuring levels of beta-amyloid noninvasively has been attempted by the targeted imaging of senile plaques. This approach fails as a specific measure of soluble A-beta peptide because current A-beta targeted imaging agents are directed at insoluble aggregates that are characteristic of A-beta fibrillar deposits in the brain. Further, targeted imaging of plaques may not provide early diagnosis, as large plaque burden is mostly associated with mid to late stage disease. Moreover, it has not been shown that current anti-A-beta therapies will affect fibrillar deposits appreciably to detect by imaging techniques at clinically relevant time points. Alternatively, in vitro measures of A-beta may be specific for soluble A-beta in the cerebral spinal fluid, but lacks the necessary selectivity for local A-beta in the brain that is necessary for direct, accurate assessment of brain levels of soluble A-beta species. To date, the targeted non-invasive measurement and imaging of soluble A-beta peptide species (including monomer, dimers, trimers and n-oligomers) that exist in the central nervous system ("CNS") have not been addressed.

Unlike the presently described imaging agents that bind to soluble A-beta, there are imaging agents and dyes that bind exclusively to insoluble deposits of A-beta or senile plaques. Small molecules that specifically bind to insoluble A-beta deposits include, for example, small molecular weight molecules, such as Congo red, Chrysamine G, methoxy-X04, TZDM, [¹¹C]6, IMSB, Thioflavin(e) S and T, TZDM, 1-BTA, benzathiozole derivatives, [¹²⁵I]3, BSB, IMSB, styrylbenzene-derivatives, IBOX, benzoxazole derivatives, IMPY, pyridine derivatives, DDNP, FDDNP, FENE, dialkylaminonaphthyl derivatives, benzofuran derivatives, and derivatives thereof (see, e.g., U.S. Pat. Nos. 6,133,259; 6,168,776; 6,114,175).

Nucleic acid sequences and derivatives thereof have been shown to bind to insoluble senile plaques of A-beta, including mRNA for furin and amyloid precursor protein ("APP").

Peptides also have been developed as imaging agents for insoluble deposits of A-beta and senile plaques. The sequence specific peptides that have been labeled for the purpose of imaging insoluble A-beta includes the labeled A-beta peptide itself, putrescine-gadolinium-A-beta peptide, radiolabeled A-beta, [¹¹¹In]A-beta, [¹²⁵I]A-beta, A-beta labeled with gamma emitting radioisotopes, A-beta-DTPA derivatives, radiolabeled putrescine, KVLFF-based ligands and derivatives thereof.

Inhibitors of aggregated A-beta have been suggested to disrupt the formation of these aggregates by interacting with soluble and/or insoluble fibrils of A-beta. Examples of inhibitors or anti-aggregation agents include peptides of A-beta, KVLFF-based ligands, small molecular weight compounds, carbon nanostructures, rifamycin, IDOX, acridone, benzofuran, apomorphine, and derivatives thereof.

Agents have also been know to promote aggregation agents such as A-beta42, proteins, metals, small molecular weight compounds, and lipids. Agents that either promote aggregation or disaggregation of A-beta fibrils presumably interact with either soluble or insoluble A-beta or both, suggesting that developing compounds that exclusively bind A-beta is feasible.

Antibodies for A-beta are similar to KLVFF-derivative as they also interact with soluble and insoluble A-beta. Antibodies specific for soluble and insoluble A-beta can be prepared against a suitable antigen or hapten comprising the desired target epitope, such as the junction region consisting of amino acid residues 13-26 and/or the carboxy terminus consisting of amino acid residues 33-42 of A-beta. One suitable antibody to soluble A-beta is disclosed in Kayed, et al., Science, vol. 300, page 486, April 18, 2003. Synthetic peptides can also be prepared by conventional solid phase techniques, coupled to a suitable immunogen, and used to prepare antisera or monoclonal antibodies by conventional techniques. Suitable peptide haptens typically will comprise at least five contiguous residues within A-beta and can include more than six residues. Synthetic polypeptide haptens can be produced by the Merrifield solid-phase synthesis technique in which amino acids are sequentially added to a growing chain (Merrifield (1963) J. Am. Chem. Soc. 85:2149-2156). Suitable antibodies include, for example, those of US Pat. Nos. 5,811,310; 5,750,349; and 5,231,000, R1282, 21F12, 3D6, FCA3542, and monoclonal and polyclonal antibodies for A-beta 1-40, 1-42 and other isoforms. Certain imaging agents have been developed that can report on the specific presence of a target molecule without binding to that molecule. In such instances the imaging agents are considered "activatable" because their signal is activated or unactivated based on the presence of a specific target molecule. Examples of such agents have been used for MRI and optical imaging (Li WH, Parigi G, Fragai M, Luchinat C, Meade TJ, Inorg Chem 2002 Jul 29;41(15):4018-24)( Louie AY, Huber MM, Ahrens ET, Rothbacher U, Moats R, Jacobs RE, Fraser SE, Meade TJ. Nat Biotechnol 2000 Mar; 18(3):321-5) (Weissleder R, Tung CH, Mahmood U, Bogdanov A Jr Nat Biotechnol 1999 Apr;17(4):375-8).

The agents described above target either soluble or insoluble (i.e., fibrillar) A-beta. Agents may bind other proteins that aggregate to form amyloid in the central nervous system or peripherally in the body of a mammal. Diseases with amyloidogenic peptides may be called amyloidoses. Amyloidosis affecting the body other the CNS are referred to as systemic amyloidosis whereas amyloidoses in the CNS include alpha-synuclein protein in Parkinson's disease, multiple myeloma-associated protein in amyloidomas, transthyretin in oculoeptomeningeal A and Meningocerebrovascular A, A-beta peptide in Down's syndrome and hereditary cerebral hemorrhage with amyloidosis (HCHWA)-Dutch type, cystatin C in HCHWA-Icelandic type, and prion protein in transmissible spongiform encephalophathy.

WO 02/16333 discloses imaging agents based on thioflavin for the *in vivo* imaging of Alzheimer's diseases and amyloid deposition. The agents comprise indoles, benzimidazoles or benzisoxazoles, which are labelled with radiohalogens or radiometals (such as ^{99m}Tc) attached *via* chelating agents.

Howlett et al [Biochem.J., 340(1), 283-289 (1999)] describe the inhibition of fibril formation in β-amyloid peptide by a series of aryl-substituted benzofurans.

WO 02/085903 discloses amyloid plaque imaging agents based on imidazoles having a fused phenyl or heterocyclic ring, and labelled with a radiohalogen such as ¹²³I, ¹⁸F or ⁷⁷Br.

WO 03/051859 discloses benzofurans having a 2-phenyl substituent and a -(CO)-phenyl substituent in the 3-position, and further comprising a chelate conjugated thereto. The chelator-benzofuran conjugates are suitable for radiolabelling with ^{99m}Tc for radiopharmaceutical imaging of a range of medical conditions *in vivo.*

Gassman et al [J.Am.Chem.Soc., 86, 5495-5508 (1974)] describe a general synthesis of indoles.

Johnson et al [J.Org.Chem., 51, 5040-5041 (1986)] disclose a synthesis of 2,3-disubstituted benzofurans, thianapthalenes and indoles *via* metallation-induced cyclisation of alkynes.

Przewoski et al [Bull.Acad.Sci.USSR, 38(3.2), 579-580 (1989)] disclose a synthesis of thiophenes *via* the reaction of sulfur with arylhaloalkanes at 200-240 °C.

### SUMMARY.

The present invention provides an imaging agent which comprises a benzofuran as defined in Formula II (below), labeled with a label chosen from radioisotopes, paramagnetic particles and optical particles.

In another aspect, methods of detecting at least one of A-beta species and amyloidogenic peptides comprising the steps of providing a sample suspected of comprising A-beta species or amyloidogenic peptides, applying an imaging agent comprising a compound described in Formula II, and detecting an amount of imaging agent bound to the at least one of A-beta species and amyloidrogenic peptides.

In another aspect, the imaging agent of Formula II for use in methods of assessing an amyloid -related disease comprising the steps of administering to a subject said imaging agent and detecting the imaging agent bound to at least one of A-beta species and amyloidogenic peptides.

In yet another aspect, the imaging agent of Formula II for use in methods of non-invasively assessing the therapeutic efficacy of therapies in a subject are described which include the steps of administering to a subject a first dose of a composition described in Formula II, and non-invasively obtaining a baseline measurement of the imaging agent within the subject, administering to the subject a therapy to be evaluated, administering to the subject a second dose of said composition, non-invasively obtaining a second measurement of the imaging agent within the subject, and comparing the two or more measurements separated in time, wherein an increase or decrease in the amount of the imaging agent present indicates the efficacy of the therapy.

### DETAILED DESCRIPTION

The present disclosure relates to a method of non-invasively assessing levels of soluble A-beta to diagnose amyloid-related diseases, including Alzheimer's disease. This method qualitatively and quantitatively determines soluble A-beta levels in vivo. This method can also be used to determine the efficacy of related therapies used for amyloid-related diseases. To assess the soluble A-beta levels, a labeled diagnostic imaging agent is delivered to a subject. Typically, the subject is a mammal and can be human. The labeled imaging agent contains a benzofuren compound that binds to soluble A-beta and a chemical entity that emits a signal detectable by an imaging modality. The labeled imaging agent is delivered to a subject by a medically appropriate means. After allowing a clearance time according to the label chosen, the amount of imaging agent bound to soluble A-beta is determined by noninvasively measuring the emitted signal using an imaging modality. The visual and quantitative analyses of the resulting images provide an accurate assessment of the global and local levels of soluble A-beta in the brain.

"A-beta species" herein refers to A-beta soluble monomers, soluble oligomers, and insoluble fibrils. "Amyloidogenic peptides" herein refer to peptides or proteins that have underwent or have the propensity to undergo an amyloidogenic process to form aggregates called amyloid, which have a secondary structure of cross-beta sheets, are birefrigent under polarized light, stain with the histological stain Congo red, and are fibrillar in nature.

The present disclosure also relates to a method of labeling and detecting A-beta species and amyloidogenic peptides as well as quantitatively measuring the amount of A-beta species and amyloidogenic peptides in vitro, ex vivo, and in situ. The agent that binds to A-beta species and amyloidogenic peptides is detected by the agent's emitted signal, such as emitted radiation, fluorescence emission, and optical properties of the agent. A-beta species and amyloidogenic peptides from at least cell culture, post-mortem human tissue, animal models of disease, and synthetic and recombinant sources are typically exposed to excess agent for a period of incubation. Agent that is nonspecifically bound or free in the incubation solution is blocked or washed away to leave agent specifically bound to A-beta species and amyloidogenic peptides that is detectable with common microscopic, widefield imaging, radiometric, fluorescent, optical, and analytical techniques. The detectable signal may be converted to numerical values to quantify the amount of targeted A-beta species and amyloidogenic peptides.

The benzofuren entity of the imaging agent that binds to soluble A-beta can bind to monomers, dimers, trimers and/or oligomers comprised of a larger number of A-beta peptides up to 24 A-beta peptides. More specifically, the soluble A-beta species to which the imaging agent can bind include monomers, dimers, trimers, and oligomers of A-beta 1-38, A-beta 1-39, A-beta 1-40, A-beta 1-41, A-beta 1-42, A-beta 1-43 or any combination thereof. The A-beta peptide in soluble monomer or oligomer forms can be derived ex vivo, by recombinant means, or synthetically. The soluble A-beta includes monomeric and low oligomeric A-beta that is soluble in an aqueous solution. In some embodiments, the soluble A-beta is of a type that remains in the supernatant of aqueous solution after centrifugation at 15000 times gravity. In some embodiments, the soluble A-beta includes A-beta monomers and its aggregates that do not exhibit green birefringence when stained by Congo red.

In one embodiment, certain compounds and their derivatives are useful as imaging agents that bind to soluble A-beta. The compositions described herein exhibit nanomolar affinity to soluble A-beta, as determined by fluorescence binding assays.

Suitable imaging agents of the present invention comprise a benzofuran compound of Formula (II): 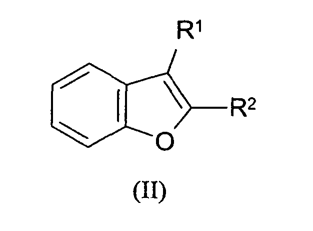 wherein

R¹ is alkyl hydroxy; and R² is a hydrocarbon radical selected from structures 1a to 53a

In one embodiment, R¹ is C₁-C₁₀ alkyl hydroxy.

The term "alkyl" as used in the various embodiments of the present invention is intended to designate both linear alkyl, and branched alkyl. The term "alkyl" also encompasses that alkyl portion of alkoxide groups. In various embodiments normal and branched alkyl radicals include as illustrative nonlimiting examples C₁-C₃₂ alkyl optionally substituted with one or more groups selected from C₁-C₁₂ alkyl,

Some particular illustrative examples comprise methyl, ethyl, n-propyl, isopropyl, n-butyl, sec-butyl, tertiary-butyl, pentyl, neopentyl, hexyl, heptyl, octyl, nonyl, decyl, undecyl and dodecyl.

R² is as defined in Table 1 below.

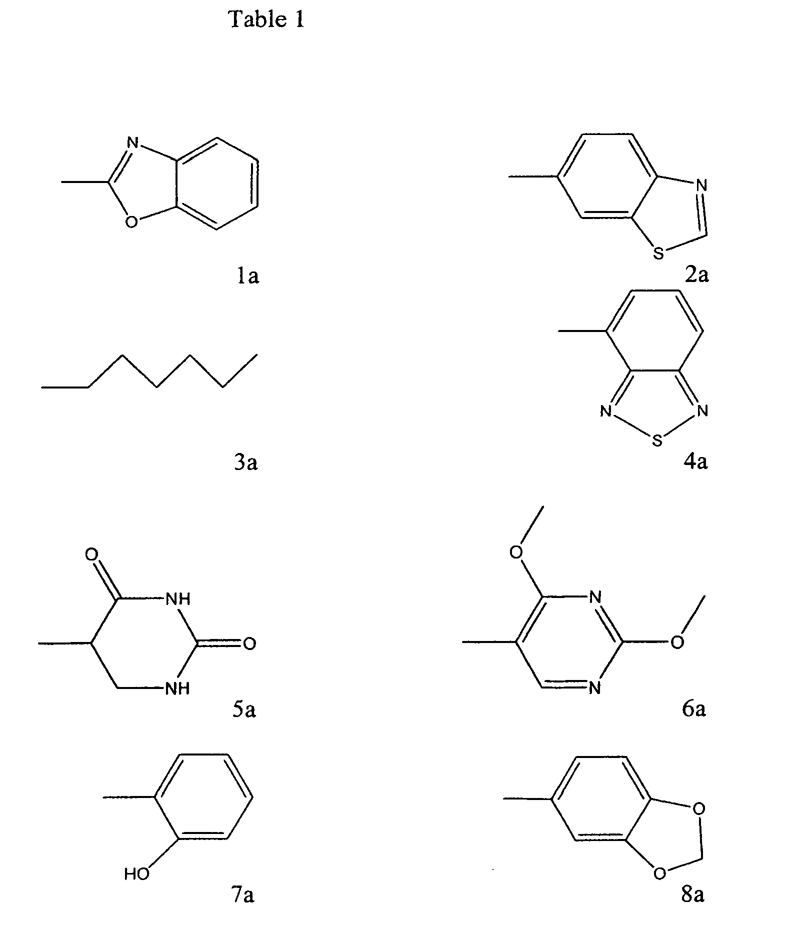

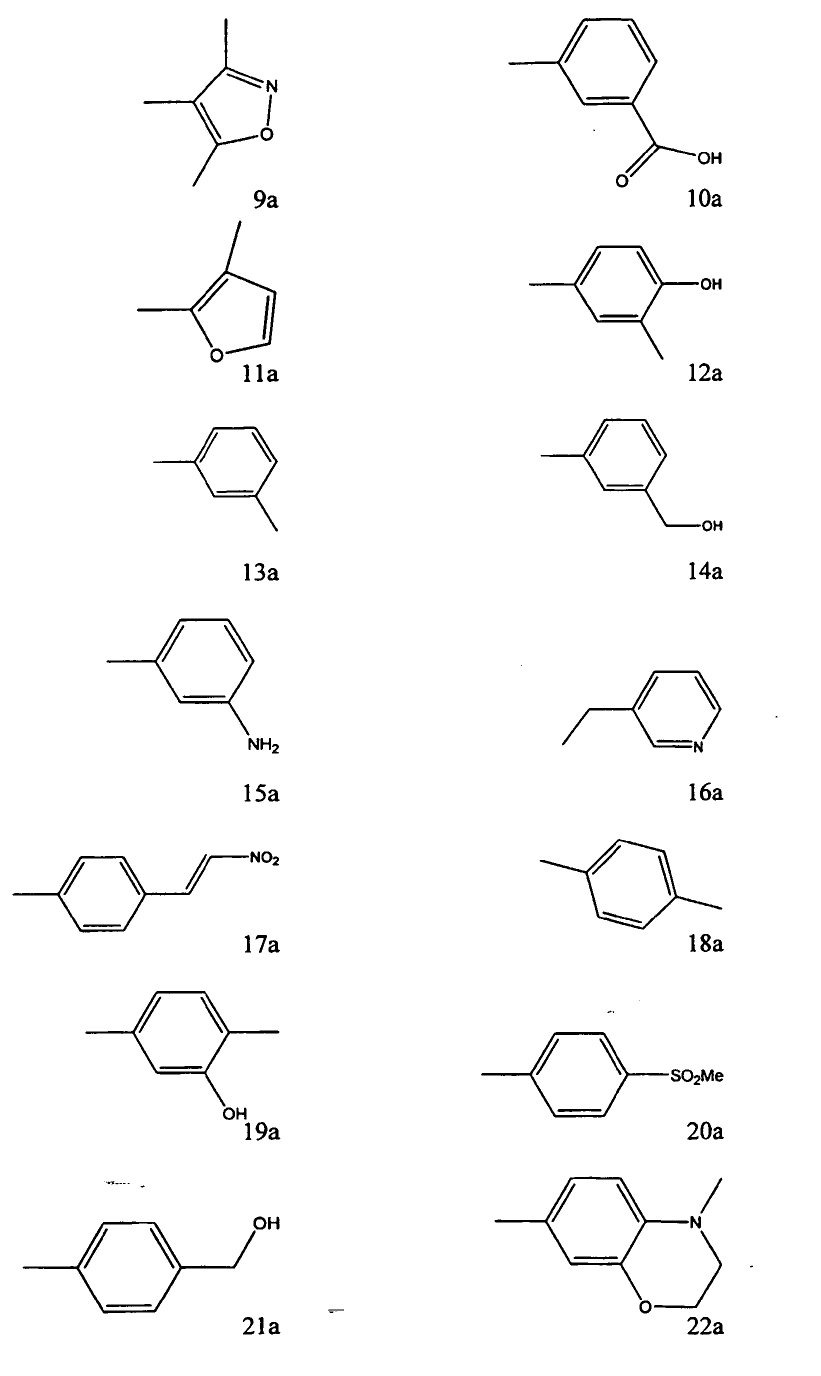

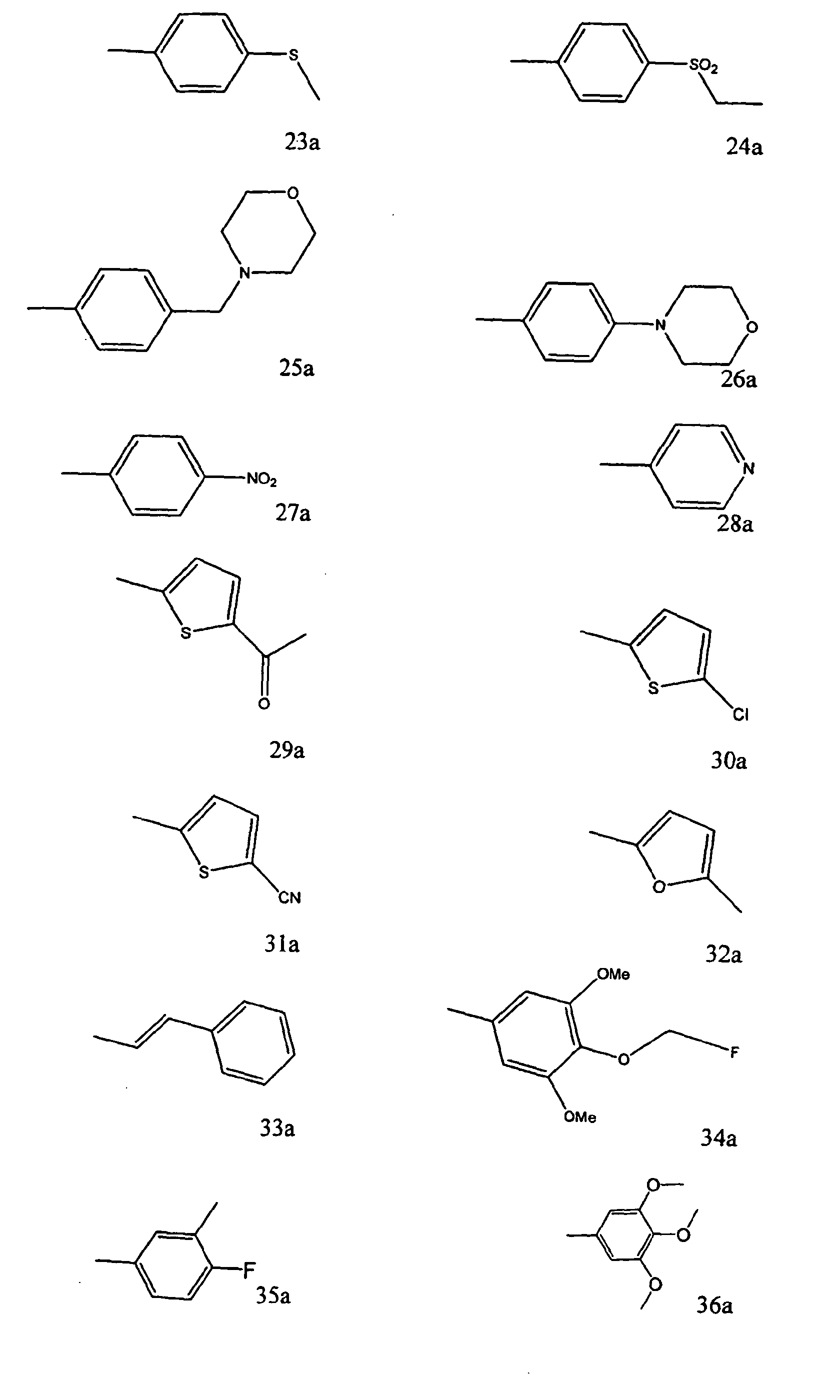

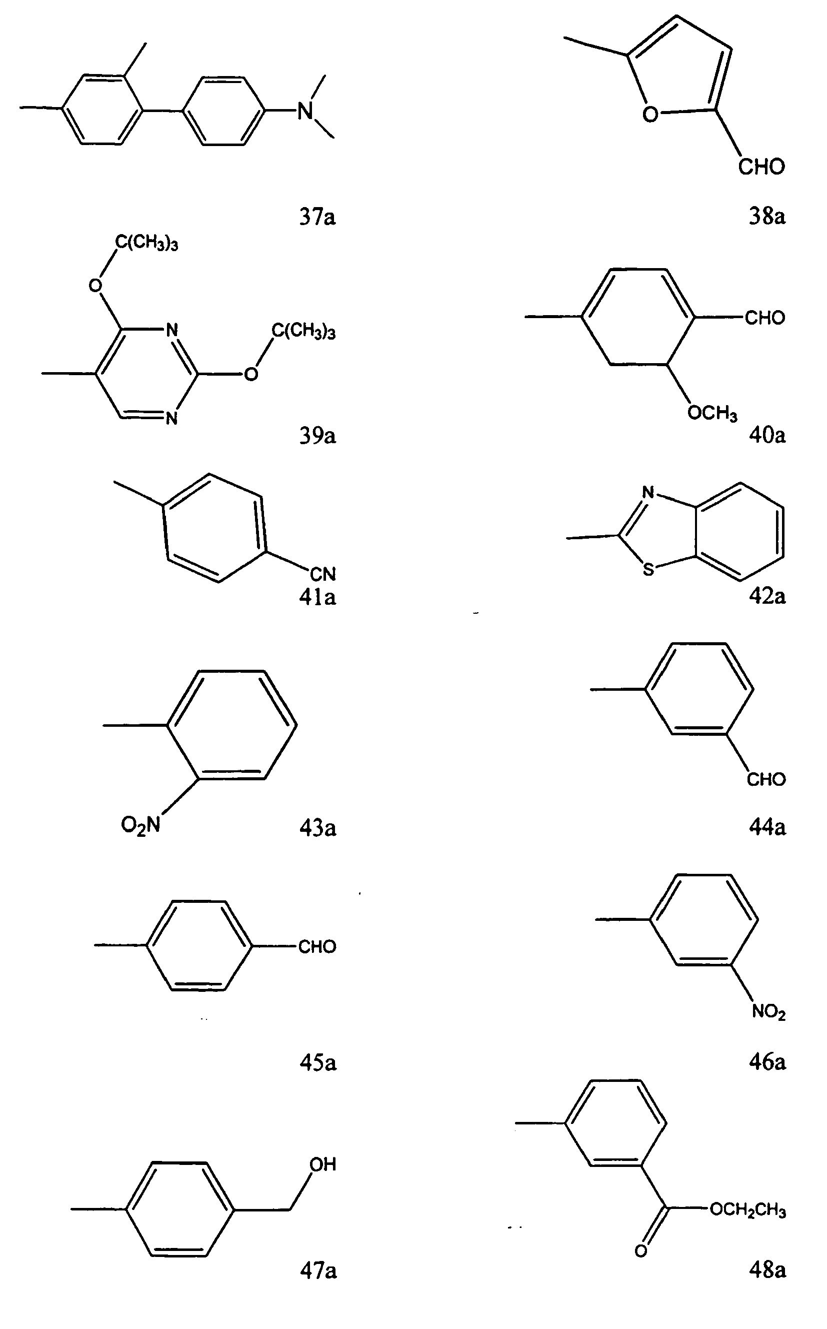

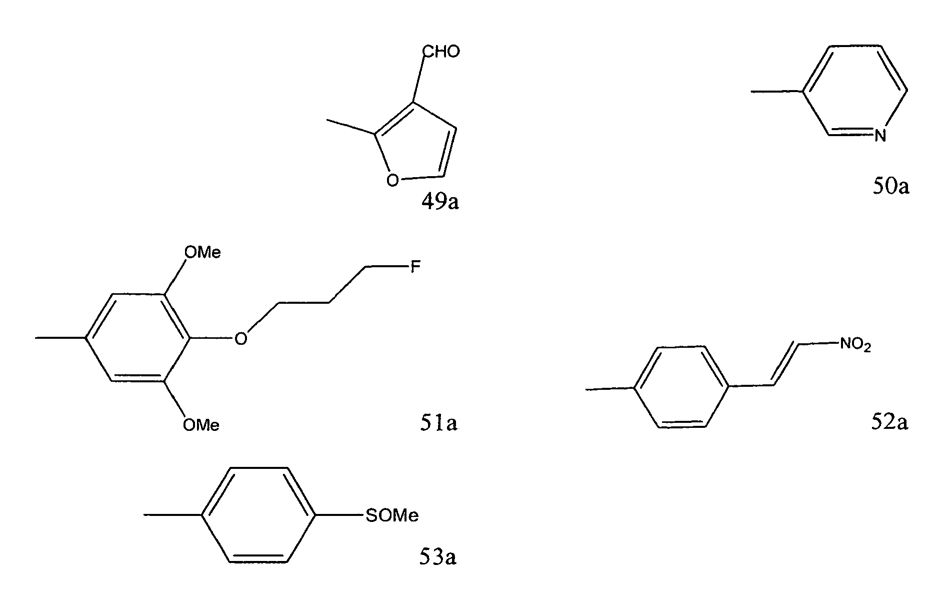

More particularly, certain benzofuran derivatives having a formula as described in Table 2 are useful as imaging agents for A-beta.

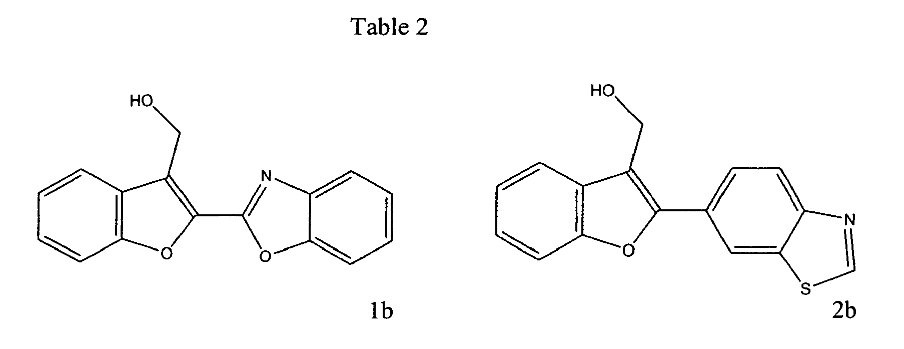

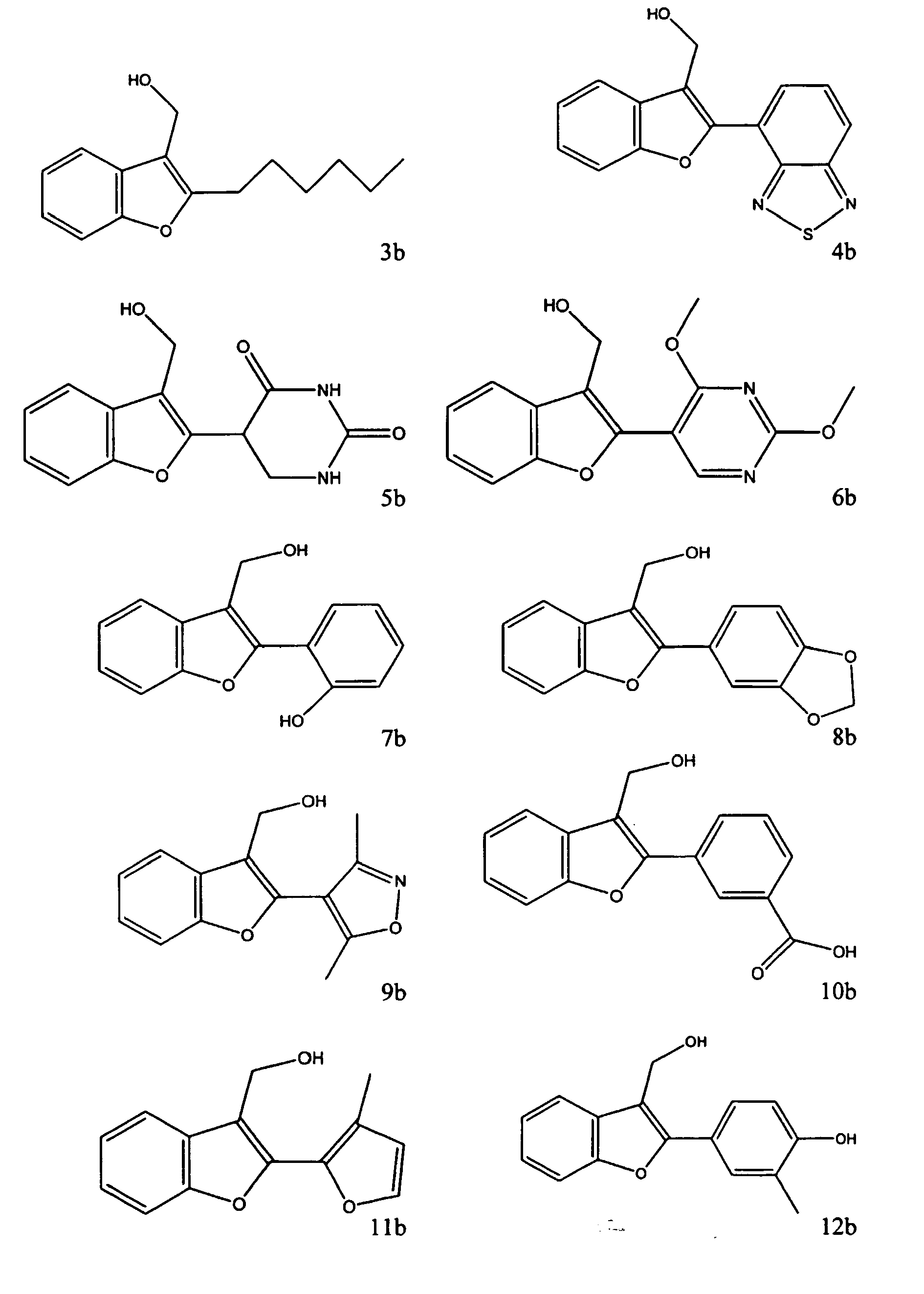

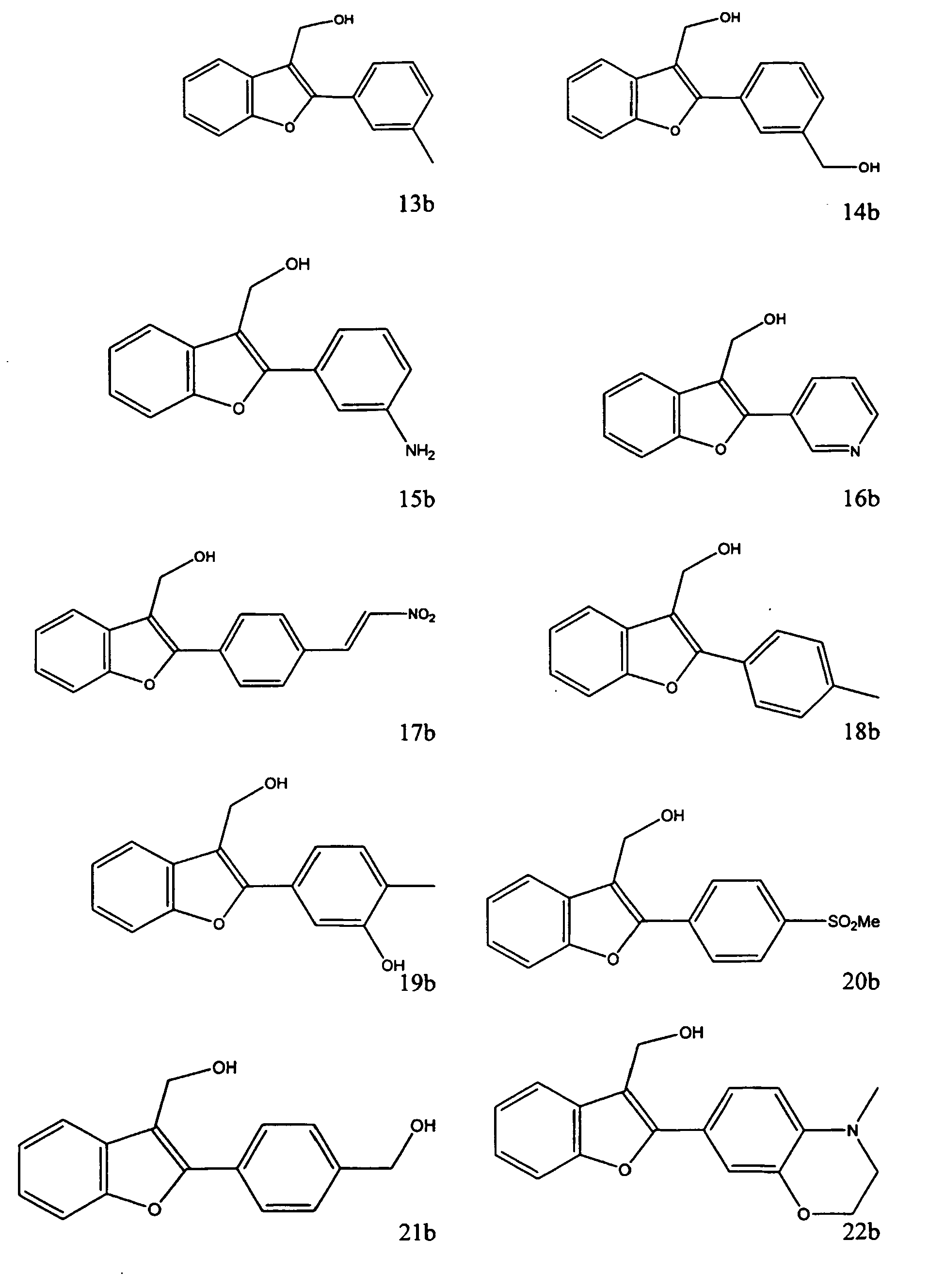

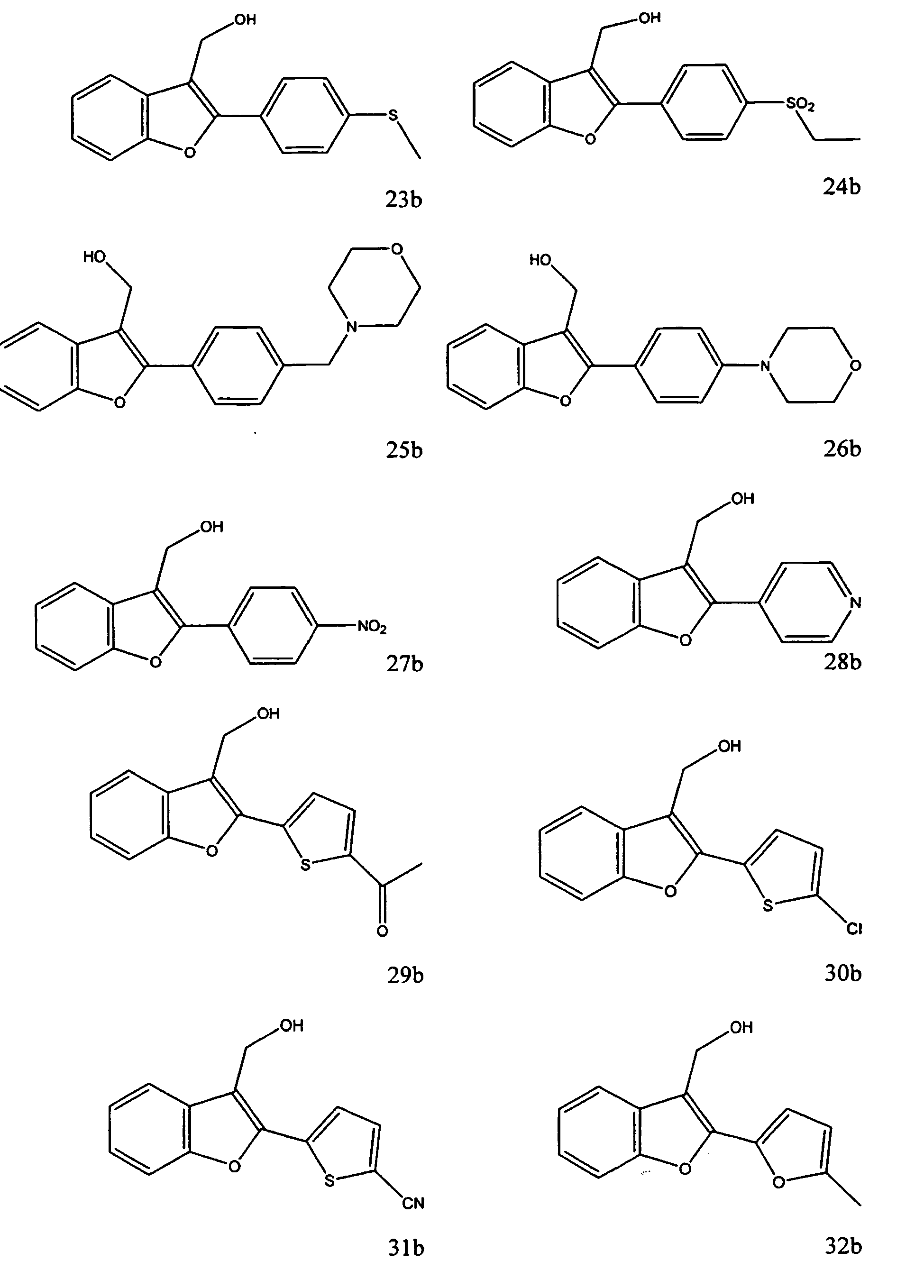

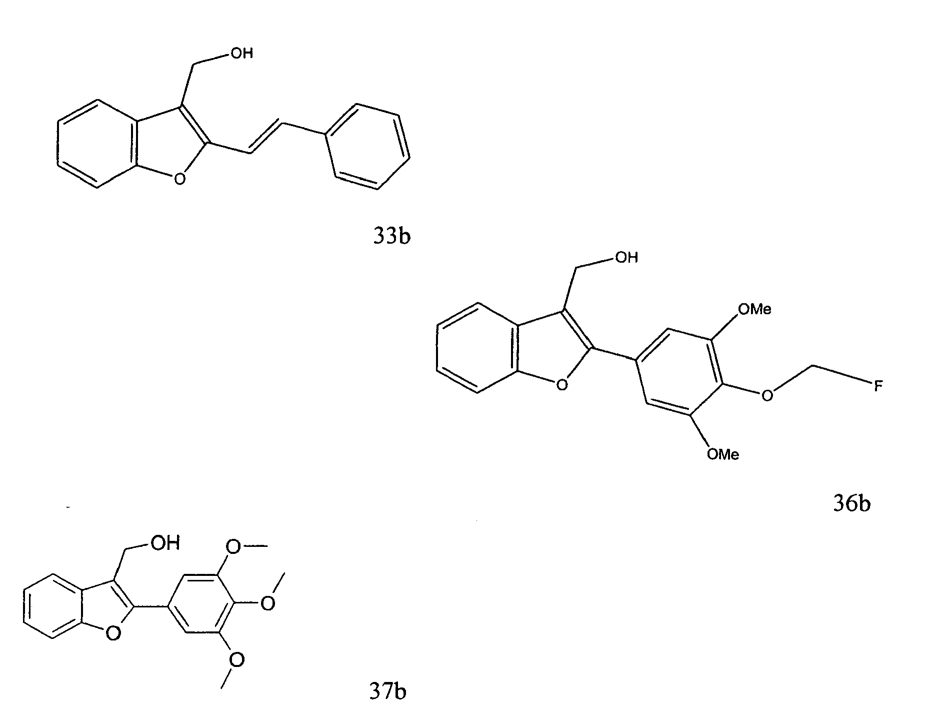

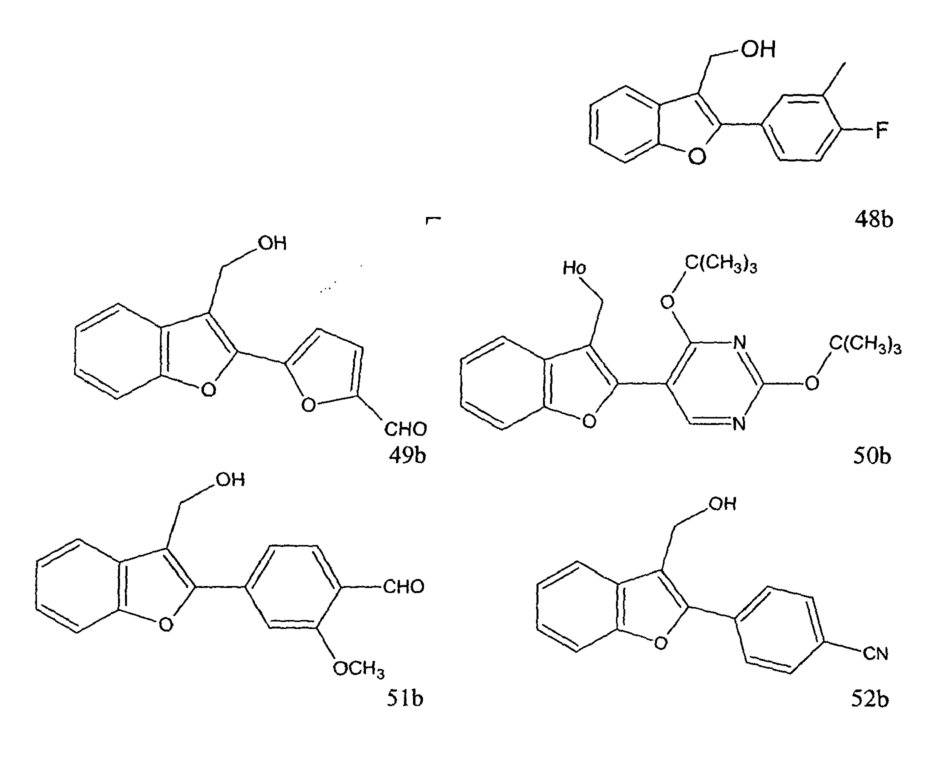

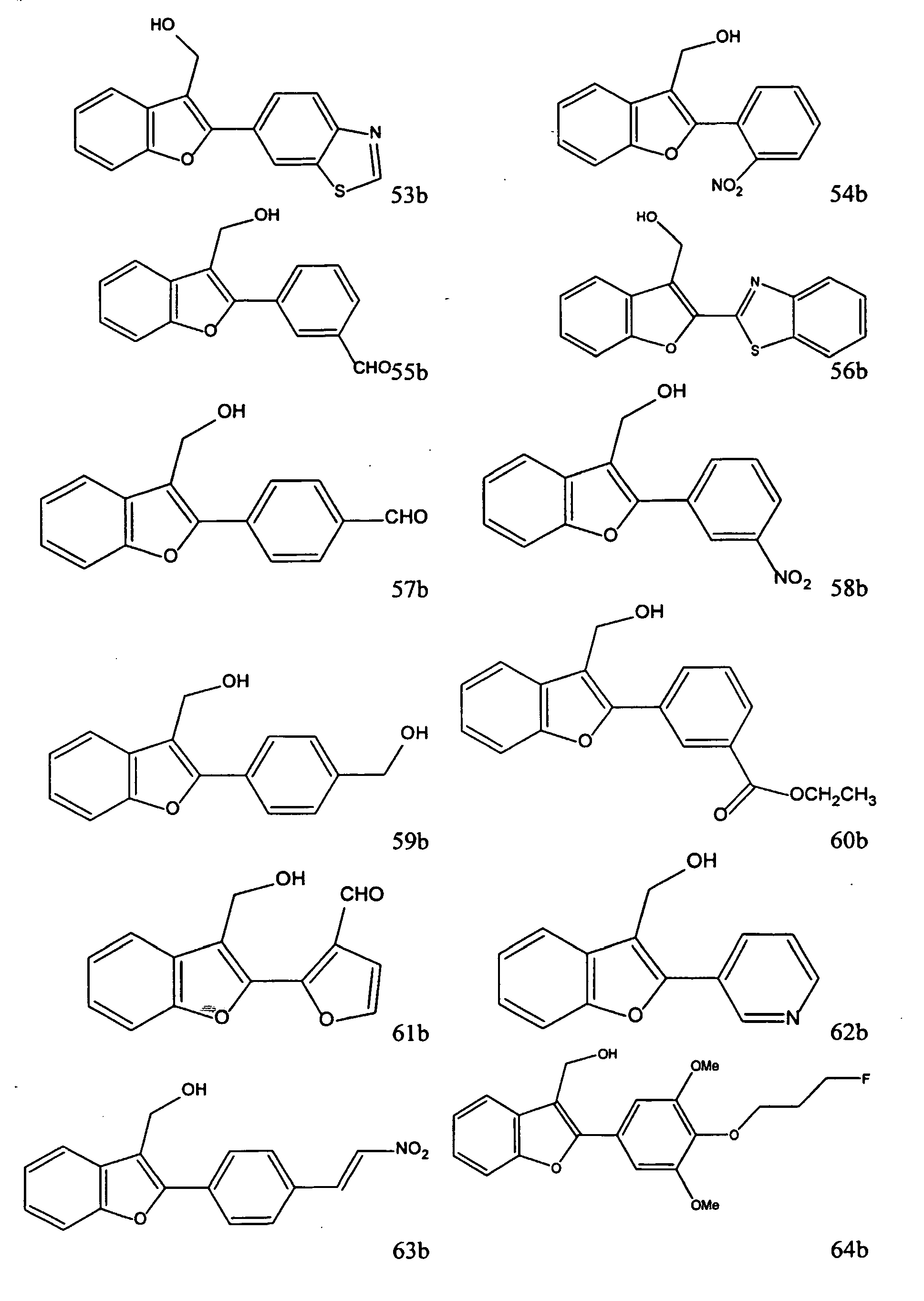

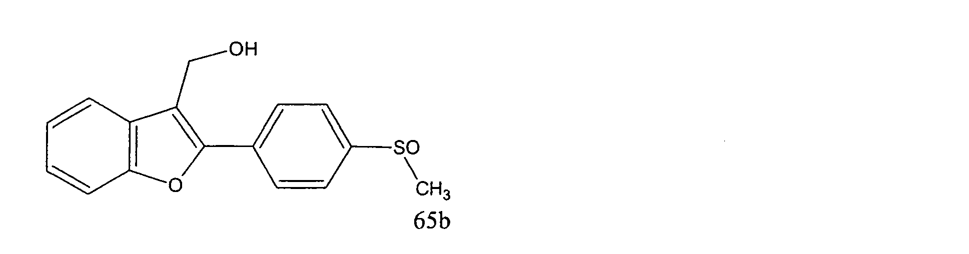

wherein "Et" is ethyl and "Me" is methyl. Substituted 2-phenyl-3-alkyl benzofurans similar to those shown in Formula II have been synthesized by the Lewis acid - mediated condensation of benzyl with phenols and aryl ethers, Palladium - catalyzed cross-coupling of benzyl ketones and α,β-unsaturated carbonyl and phenolic compounds with o-dibromobenzenes, and McMurry-type reductive cyclization of dicarbonyl compounds catalyzed by low-valent Ti.

Pd-catalyzed cyclizations are also the preferred method for the synthesis of a variety of 2-alkylbenzofurans and 2-arylbenzofurans. These methods, however, are not versatile enough to allow for the synthesis of a large variety of benzofurans of the general structure shown. A more versatile and general approach is depicted below. Using the 2-bromo-3-formylbenzofuran as a starting point, reaction with commercially available organozinc reagents leads to the introduction of functionalized moieties at the 3-position. Suzuki coupling using the 2-bromo-functionality allows for the introduction of functionalized 2-arylsubstituents, in the presence of a variety of functional groups. These two steps could be interchanged, if practical advantages arise. The use of microwave accelerated organic synthesis allows for the rapid evaluation of synthetic strategies and optimization of reaction conditions before molecular diversity through high throughput organic synthesis is pursued. The formyl substituent permits an efficient entry to functionalized amine moieties via a reductive amination sequence.

The chemical entity of an imaging agent, of Formulas II, that emits a detectable signal (also called a label) can be either inherent or a radiolabel, a paramagnetic label, an optical label and the like. "Label" is used hereinafter to include both inherent labels, such as the agents' own fluorescence or chemiluminescence, or a label added to the agent. The type of imaging modality available will be an important factor in the selection of the label used for an individual subject. For example, a radiolabel must have a type of decay that is detectable by the available imaging modality. Suitable radioisotopes are well known to those skilled in the art and include beta-emitters, gamma-emitters, positron-emitters, and x-ray emitters. Suitable radioisotopes include ³H, ¹¹C, ¹⁴C, ¹⁸F, ³²P, ³⁵S, ¹²³I, ¹²⁵I, ¹³¹I, ⁵¹Cr, ³⁶CI, ⁵⁷Co, ⁵⁹Fe, ⁷⁵Se and ¹⁵²Eu. Isotopes of halogens (such as chlorine, fluorine, bromine and iodine), and metals including technetium, yttrium, rhenium and indium are also useful labels. Typical examples of metallic ions which can be bound are ^{99m}Tc, ¹²³I, ¹¹¹In, ¹³¹I, ⁹⁷Ru, ⁶⁷Cu, ⁶⁷Ga, ¹²⁵I, ⁶⁸Ga, ⁷²As, ⁸⁹Zr, and ²⁰¹Tl. For use with the present disclosure, radiolabels can be prepared using standard radiolabeling procedures well known to those skilled in the art. Preferably, the labeling will be accomplished by incorporation of one of the above-listed labels into one or both of the R¹ or R² groups.

The disclosed compounds can be radiolabeled either directly by incorporating the radiolabel directly into the compounds or indirectly by incorporating the radiolabel into the compounds through a chelating agent, where the chelating agent has been incorporated into the compounds. Such radiolabeling should also be reasonably stable, both chemically and metabolically, applying recognized standards in the art. Also, although the label can be incorporated in a variety of fashions with a variety of different radioisotopes, such radiolabeling should be carried out in a manner such that the high binding affinity and specificity of the unlabeled binding moiety is not significantly affected.

Preferred radioisotopes for in vivo diagnostic imaging by positron emission tomography ("PET") are ¹¹C, ¹⁸F, ¹²³I, and ¹²⁵I, with ¹⁸F being the most preferred. Typically, the labeled atom is introduced to the labeled compounds at a late stage of the synthesis. This allows for maximum radiochemical yields, and reduces the handling time of radioactive materials. When dealing with short half-life isotopes, an important consideration is the time required to conduct synthetic procedures, and purification methods. Protocols for the synthesis of radiolabeled compounds are described in Tubis and Wolf, Eds., "Radiopharmacy", Wiley- Interscience, New York (1976); Wolf, Christman, Fowler, Lambrecht, "Synthesis of Radiopharmaceuticals and Labeled Compounds Using Short-Lived Isotopes", in Radiopharmaceuticals and Labeled Compounds, Vol. 1, p. 345-381 (1973).

Paramagnetic labels can be metal ions are present in the form of metal complexes or metal oxide particles. Suitable paramagnetic isotopes include ¹⁵⁷Gd, ⁵⁵Mn, ¹⁶² Dy, ⁵²Cr, and ⁵⁶Fe. The paramagnetic label can be attached to the binding moiety by -several approaches. One approach is direct attachment of one or more metal chelators to the binding moiety of the imaging agent. Alternatively, the binding portion of the imaging agent can be attached to a paramagnetic metal ion or heavy atom containing solid particle, or to an echogenic gas microbubble. A number of methods can be used to attach imaging agent, which specifically binds to soluble A-beta, to paramagnetic metal ion or heavy atom containing solid particles by one of skill in the art of the surface modification of solid particles. In general, the imaging agent is attached to a coupling group that reacts with a constituent of the surface of the solid particle. The coupling groups can be any of a number of silanes, and also include polyphosphonates, polycarboxylates, polyphosphates or mixtures thereof, which react with surface hydroxyl groups on the solid particle surface, as described, for example, in U.S. patent application publication 2002/0159947 and which can couple with the surface of the solid particles, as described in U.S. Pat. No. 5,520,904.

The imaging agent itself can be fluorescent or can be tagged with optical labels that are fluorophores, such as fluorescein, rhodamine, Texas Red, and derivatives thereof and the like. The labels can be chemiluminescent, such as green fluorescent protein, luciferin, dioxetane, and the like. These fluorophore probes are commercially available, e.g., from Molecular Probes, Inc., Eugene, Oregon. The imaging agent that binds to soluble A-beta can be linked to the portion of the compound that emits a detectable signal by techniques known to those skilled in the art.

The labeled imaging agent can typically be administered to a patient in a composition comprising a pharmaceutical carrier. A pharmaceutical carrier can be any compatible, non-toxic substance suitable for delivery of the labeled A-beta binding compound to the patient, including sterile water, alcohol, fats, waxes, proteins, and inert solids may be included in the carrier. Pharmaceutically acceptable adjuvants (buffering agents, dispersing agent) can also be incorporated into the pharmaceutical composition. Carriers can contain a solution of the imaging agent or a cocktail thereof dissolved in an acceptable carrier, preferably an aqueous carrier. A variety of aqueous sterile carriers can be used, e.g., water, buffered water, 0.4% saline, 0.3% glycine, 25% human serum albumin and the like. The solutions are preferably pyrogen-free, sterile, and generally free of particulate matter. The compositions can contain additional pharmaceutically acceptable substances as necessary to approximate physiological conditions such as pH adjusting and buffering agents, toxicity adjusting agents and the like, for example sodium acetate, sodium chloride, potassium chloride, calcium chloride, sodium lactate. The concentration of imaging agent in the composition solutions may vary as required. Typically, the concentration will be in trace amounts to as much as 5% by weight depending on the imaging modality and are selected primarily based on fluid volumes, and viscosities in accordance with the particular mode of administration selected. Preferably, the concentration is between about 0.1 and about 1 nmol, more preferably from about 0.1 nmol to about 0.5 nmol. The imaging agent may be present in several ml of injectable solution, as would be determined based on dose, and easily calculated by one of ordinary skill in the art. If the agent is labeled with ¹⁸F, approximately 105 pmol of ¹⁸F yields 10 mCi of a radiation dose initially. This amount of radioactivity is typical and considered safe in the current medical imaging procedures. A typical composition for intravenous infusion can be made to contain 250 ml of sterile Ringer's solution and up to 100 mg, preferably around 10 mg, of the imaging agent. The composition containing the imaging agent can be combined with a pharmaceutical composition and can be administered subcutaneously, intramuscularly or intravenously to patients suffering from, or at risk of, amyloid-related conditions.

The imaging agent is administered to a subject to determine the presence and amount of soluble amyloid in the subject. After administration, clearance time can, if desired, be permitted which allows the imaging agent to travel throughout the subject's body and bind to any available soluble A-beta whereas the unbound imaging agent passes through the subject's body. In a case where the imaging agent does not directly bind, but rather reports on the presence of the A-beta, sufficient time is allowed for a specific interaction to occur in which the reporter molecule is "activated". The clearance time will vary depending on the label chosen for use and can range from 1 minute to 24 hours. The imaging agent is then detected noninvasively in the subject's body by an imaging modality. The imaging modality can include positron emission tomography ("PET"), optical, single photon emission computed tomography ("SPECT"), magnetic resonance imaging ("MRI"), ultrasound, computed tomography ("CT"), and the like, depending on the label used, the modality available to medical personnel and the medical needs of the subject. Equipment and methods for the foregoing imaging modulations are known to those skilled in the art.

The imaging agent can be delivered and the imaging taken to determine the amount of soluble A-beta present in the subject's body as an indication of disease or pre-disease states. The levels of soluble A-beta can be indicative of pre-disease conditions and therapies toward removal of the soluble A-beta and/or its precursors can prevent or forestall the onset of an amyloid-related disease, such as Alzheimer's disease. The removal of soluble A-beta can also improve the condition of a subject that already exhibits clinical signs of disease.

In another aspect, the present methods can be used to determine the efficacy of therapies used in a subject. By using multiple images over time, the levels of A-beta can be tracked for changes in amount and location. This method can aid physicians in determining the amount and frequency of therapy needed by an individual subject. In this embodiment, an imaging agent in accordance with the present disclosure is administered and a baseline image is obtained. The therapy to be evaluated is administered to the subject either before or after a baseline images are obtained. After a pre-determined period of time, a second administration of an imaging agent in accordance with their disclosure is given. A second or more images are obtained. By qualitatively and quantitatively comparing the baseline and the second image, the effectiveness of the therapy being evaluated can be determined based on a decrease or increase of the signal intensity of the second image or additional images.

The following nonlimiting Examples are shown and describe various embodiments of the present invention.

### EXAMPLE 1

Preparation of 2-bromo-3-bromomethyl benzo[b]furan: To a suspension of N-bromosuccinimide (10.8g, 60 mmol) in 3-methylbenzofuran(4g, 30.26 mmol) and carbon tetrachloride (20 ml), was added benzoyl peroxide (100 mg) and the mixture was refluxed for 3 hrs. Fresh NBS was added (1.1 g) and the mixture was refluxed for 1 hr. Following the addition of more NBS (1g) and refluxing for one more hour, GC-MS analysis of the reaction mixture indicated that the desired product was accompanied by 10% mono-brominated and 8% tri-brominated products. The succinimide was filtered off, washed with CCl₄ until white and the solvent was stripped off to give a brown, viscous oil. Absolute ethanol (12 ml) was added, the solution was cooled to -25°C and the mass of yellow crystals was filtered at -25°C with a fritted canula. The crystals were washed at -40°C with 12 ml ethanol and dried under vacuum to give the desired product as a mass of needle like crystals (95% pure by GC-MS), 7.672g (87.4%).

### EXAMPLE 2

Preparation of 2-bromo-3-hydroxymethyl benzo[b]furan (2): To a 250 ml flask was added the dibromide (7.672g, 26.45 mmol) in dioxane (30 ml), followed by a solution of NaHCO₃ in water (30 ml). The mixture was heated to reflux for one hour, while stirring it vigorously. The cooled mixture was then diluted with water (150 ml) and extracted with dichloromethane (5x). The extract was washed with brine, dried, the solvent was stripped, and the resulting orange oil was dissolved in 12 ml chloroform and cooled to -20°C. The resulting crystals were filtered as before at -40°C, washed with cold chloroform and dried under reduced pressure to give the desired product as light yellow crystalls (GC-MS purity 98%), 3.72g (62.2%).

### EXAMPLE 3

Preparation of 2-bromo-3-formyl benzo[b]furan (3): To a 50 ml round bottom flask were added powdered pyridinium chlorochromate (443 mg, 2.055 mmol), Celite^{®} (450 mg) and dry dichloromethane (20 ml). A solution of the alcohol 2 as in Formula I, wherein R¹=CH₂OH, R²=Br, X=O (226 mg, 1 mmol) in dichloromethane (1ml) was added and the mixture was stirred at r.t. in the dark for one hour. TLC (hexanes/ethyl acetate 4/1 v/v) indicated complete conversion (R_{f} 0.7 vs. R_{f} 0.3 for starting material). The mixture was diluted with ether, flushed through a 1 in plug of silicagel, the solvent was stripped and the residue was again flushed through a 1 in plug of silicagel, using hexanes/ethyl acetate 2/1 v/v. to give the desired product as light orange crystals (205 mg, 91%).

### EXAMPLE 4

General procedure for the Suzuki coupling reaction: To a microwavable vial were added the 2-bromo-3-substituted benzofuran (0.1 mmol), arylboronic acid (2 equivalents), tris(dibenzylidene acetone) dipalladium (Pd₂dba₃, 0.03 equivalents), K₂CO₃ (1.5 equivalents) and degassed dimethylformamide (1 ml). The vial was purged with N₂, capped, and irradiated for 10 minutes at 120C (initial power 50W). Water was then added (2 ml), the mixture was extracted with ether (5x), washed with water, brine, dried over Na₂SO₄ and the crude product was purified by MPLC using hexanes/ethyl acetate gradient. All reactions were analyzed for conversion prior to work up. Unless otherwise stated, cooling was not turned on during the reaction. Also, 10 minutes reaction time were generally found to result in complete consumption of the bromofuran starting material.

### EXAMPLE 5

General procedure for the addition of organometallic reagents to aldehyde 3, as in Example 3, (Formula I, R¹=CHO, R²=Br, X=O): To a dry 5 ml flask was added the aldehyde (0.2 - 1 mmol), dry ether (1.5 ml) and the mixture was cooled to 0C. The organometallic reagent solution (1.25 equivalents) was then added dropwise and the mixture was stirred at 0C for 30 minutes. At this time, GC-MS generally indicated complete conversion. The ether layer was washed with aqueous citric acid, dried and the compound was purified by MPLC using hexanes/ethylacetate gradient. When the corresponding ketone was the desired product, the crude secondary alcohol was oxidized according to the procedure outlined in Example 3.

### EXAMPLE 6

General procedure for the reductive amination of 2-aryl-3-formyl benzofurans: To a 1 ml vial was added the aldehyde (0.05 - 0.1 mmol), dichloroethane (0.4 ml), 2 equivalents of the priamry or secondary amine, 1 equivalent of AcOH and 1.5 equivalents of NaBH(Oac)3. The mixture was stirred vigorously at room temperature. Most reactions were complete in 4-10 hrs as evidenced by GC-MS analysis. The solvent was stripped with a nitrogen stream, ethyl acetate and silicagel (100-200 mg) was added and the crude product was purified by MPLC using the solid sample technique.

### EXAMPLE 7

General procedure for the preparation of 2-aryl-3-aminomethyl benzofurans:
a) To a dry vial was added triphenylphosphine (275 mg, 1.05 eq.) and phthalimide (149 mg, 1.02 eq.). The vial was stoppered and purged with N2. Dry THF (2 ml) was added followed by a solution of of the alcohol 2 (as in Formula I, R¹=CH₂OH, R²=Br, X=O) in THF (1 ml) and immediately by diisopropyl-azo-dicarboxylate (DIAD), dropwise. The mixture warmed up as the phthalimide dissolved, and the vial was cooled with an air stream. The resulting yellow solution was stirred at r.t. for 14 hrs. Silicagel was added to the reaction mixture and the solvent was stripped. The desired 2-bromo-3-(phthalimidomethyl) benzofuran was obtained in 84% yield following purification by MPLC using hexanes ethyl acetate 0-40% v/v gradient.
b) Introduction of various aryl moieties at the 2 position, using the intermediate above, proceeded smoothly following the general Suzuki coupling methodology described in Example 4.
c) Deprotection of the 2-aryl-3-benzofuranylmethyl phthalimides thus obtained was achieved following treatment of the corresponding intermediate, as a 0.1M solution in isopropanol, with 1.05 equivalents of N,N-dimethyl-1,3-propane diamine and microwave irradiation at 110C for 10 minutes. Upon addition of silicagel the solvent was stripped off and the desiread amine was obtained following MPLC using hexanes/ethyl acetate containing 0.1% triethylamine.

### EXAMPLE 8

Intermediates 34b, 35b (amides, ureas, urethanes in the compounds table) were prepared as follows: to a dry vial containing the amines (example NN1, c) (0.1 mmol in 1 ml dry dichloromethane) was added the isocyanate 1.05 eq. and the mixture was stirred at room temperature for 4 hrs or until GC-MS analysis indicated complete conversion. The solvent was stripped, the residue taken in 1 ml ethyl acetate and filtered through a 3 ml SPE cartridge (silicagel) using ethyl acetate. The product was > 95% pure (GC-MS) and was used without further purification. Similarly, acid chlorides were used for the synthesis of amides; in this case, 1 eq. of triethylamine was used as an acid quencher. This procedure was also used for the synthesis of urethanes, when 1.25 eq. of chloroformate was generally needed for the reaction to proceed to completion. Filtration through an SPE cartridge of basic alumina produced compounds of sufficient purity to be used without further MPLC purification.

### EXAMPLE 9

Soluble A-beta (1-42) formation: Soluble A-beta (1-42) (Bachem, Torrance, CA; lot # 0560840 and 0535120) was prepared by first chilling 1 mg of A-beta (1-42) and 375-500 µL of 1,1,1,3,3,3-hexafluoro-2-propanol (Sigma, St. Louis, MO; HFIP) in separate bottles on ice for 30 min. The cold A-beta (1-42) was solubilized by adding the cold HFIP and incubating the turbid A-beta -HFIP mixture for 1 hr at room temperature. The resulting clear A-beta -HFIP solution was then dried to a clear film under vacuum. The film was picked up in 22 µL of dimethyl sulfoxide (Sigma, DMSO) and then diluted with phosphate buffered saline (pH 7.4, PBS) to a volume of 1.107 ml (200 µM).

Insoluble A-beta (1-40) fibril formation: A-beta (1-40) fibrils were solubilized in HFIP and then dried to a clear film as described above for soluble A-beta (1-42) formation. The dried clear film of A-beta (1-40) was diluted to 6 mg/mL with filtered distilled, deionized water and incubated, if necessary in a sonic bath, at room temperature until a clear solution was produced. The resulting solution was further diluted with PBS (pH 7.4) to achieve a final concentration of 200 µM A-beta and then incubated in an orbital shaker at 100-200 rpm for 3-4 days at 37 °C. A cloudy solution was produced, and the fibril concentration was calculated using Congo red. Fibrils were used immediately after their production was confirmed.

Fluorescence titration assays: Fresh solutions of 2-4 mM probe in methanol were appropriately diluted with PBS (pH 7.4) to obtain assay solutions with final concentration range of 0.1 nM to 100 µM probe in 300-400 µL PBS (pH 7.4, 2% methanol) with 10 µM A-beta (1-40) fibrils or 6.6 µM soluble A-beta (1-42). A duplicate series of assay solutions were aliquoted with the volume of soluble A-beta or fibrils replaced by PBS. The set of solutions with probe and soluble/fibrillar A-beta and the duplicate set without A-beta were prepared and the fluorescence spectra measured in triplicate. Spectral changes in the probe's fluorescence emission spectra that were unique to the presence of A-beta indicated specific binding interactions between the probe and A-beta species. This unique spectral change allowed for the determination of the probe's binding affinity. Fluorescence emission spectra for binding affinity determinations are measured with fluorometric instrumentation known to those skilled in the art, including at least a spectrofluorometer and spectrograph. Equilibrium dissociation constants were calculated using a one-site saturation model of binding. Similar determinations of equilibrium dissociation constants can utilize instrumentation known to those skilled in the art to measure phenomena including at least absorbance, radioactivity, reflected light, polarized light, mass spectrometry and the like.

### EXAMPLE 10

Select benzofuran congeners exhibited nanomolar binding affinity to soluble A-beta (1-42) but no appreciable binding to A-beta (1-40) fibrils, as determined by fluorescence binding assays. A spectral change of a probe's fluorescence that is unique to the presence of soluble or fibrillar A-beta was used to calculate the binding affinity of a probe. For example, the 22 nM *K*_{D} value (Table 3) indicates the selective binding of 37b (Table 2) for soluble A-beta with a characteristic increase in fluorescence intensity (and quantum yield) approaching approximately 5 times the fluorescence intensity (and quantum yield) of 37b in 2% methanol in PBS. 37b in 10 µM of A-beta (1-40) fibrils had minimal specific binding to the fibrils as demonstrated by an absence of significant enhanced fluorescence intensity in the presence of fibrils. 39b (Table 2) is an example of a benzofuran derivative that specifically binds to A-beta (1-40) fibrils instead of soluble A-beta 42. In constrast, 38b neither binds to A-beta in soluble nor insoluble fibrillar form (Table 3).

**Table 3**

| **Compound** | ***K*_{D} of compound to soluble A-beta (1-42)** | ***K*_{D} of compound to insoluble A-beta (1-40) fibrils** |
|---|---|---|
| 38b | Nonspecific binding | Nonspecific binding |
| 39b | No binding < 100 µM | 1.68 µM |
| 37b | 22.8 nM | No binding < 100 µM |

Compounds 38b and 39b are reference compounds having the following formulae: While the invention has been illustrated and described in typical embodiments, it is not intended to be limited to the details shown, since various modifications and substitutions can be made without departing in any way from the spirit of the present invention. As such, further modifications and equivalents of the invention herein disclosed may occur to persons skilled in the art using no more than routine experimentation, and all such modifications and equivalents are believed to be within the scope of the invention as defined by the following claims.

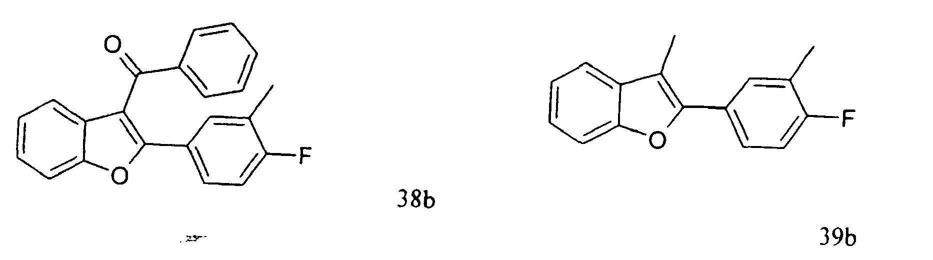

## Claims

1. An imaging agent which comprises a benzofuran compound of formula (II): 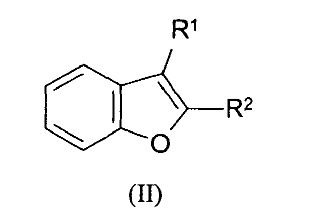 wherein R¹ is alkyl hydroxy; and R² is a hydrocarbon radical selected from the group consisting of structures 1a to 53a; 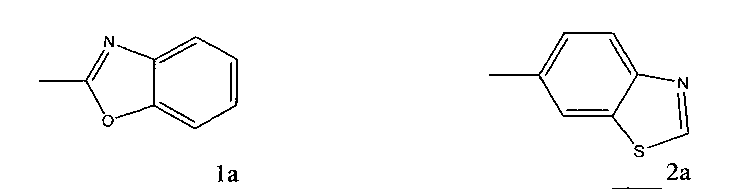 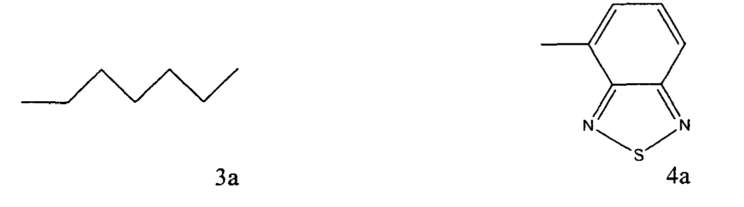 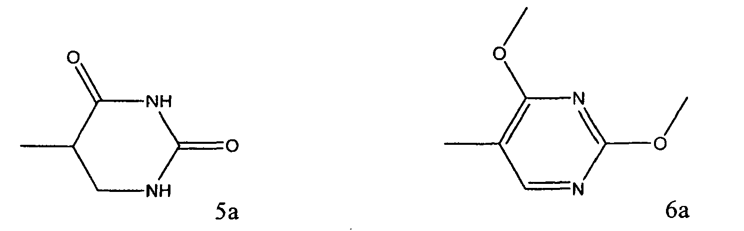 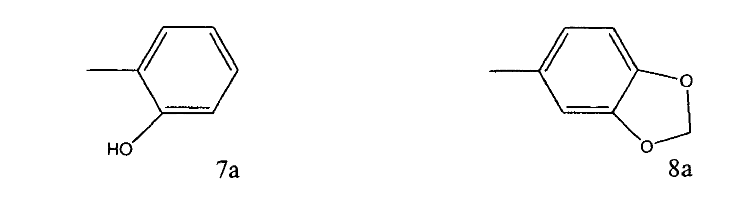 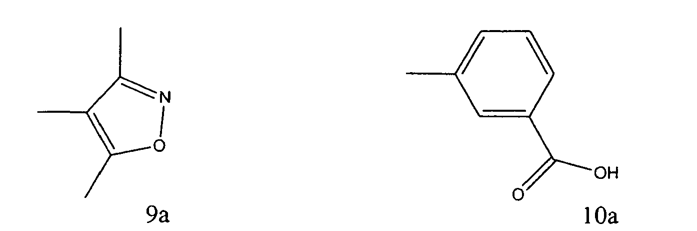 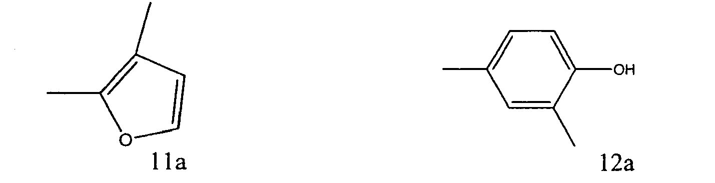 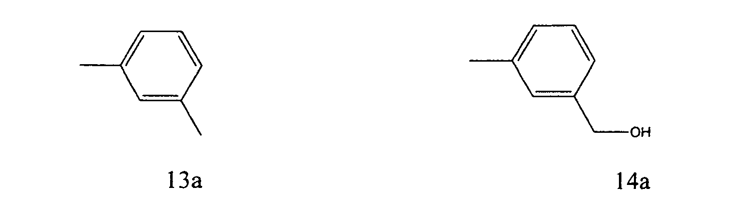 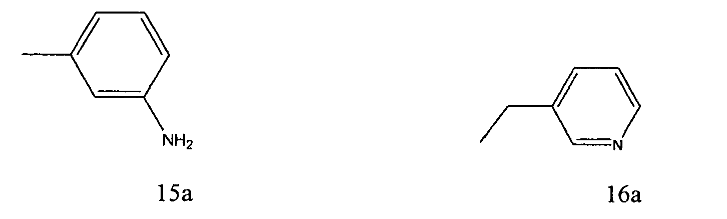 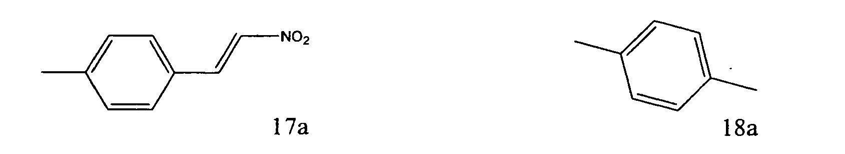 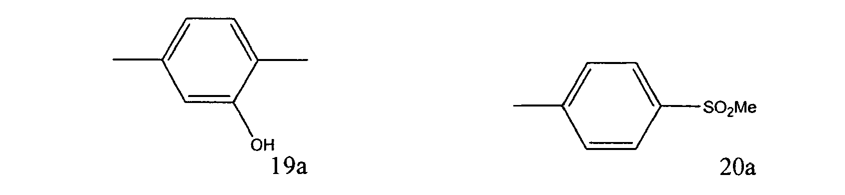 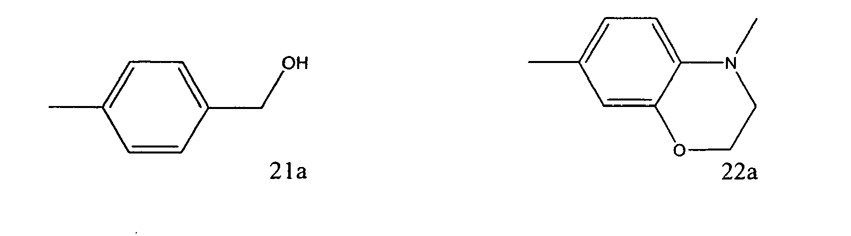 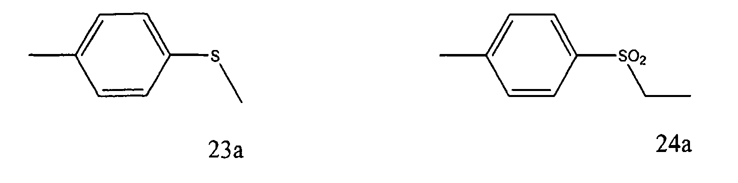 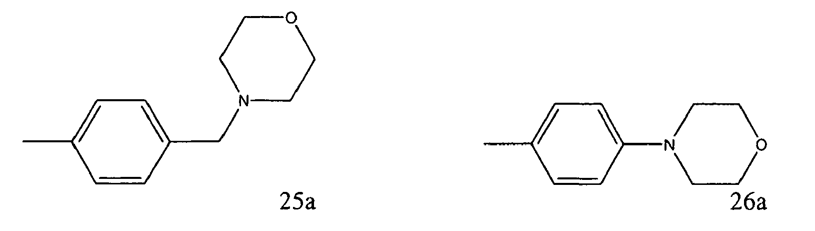 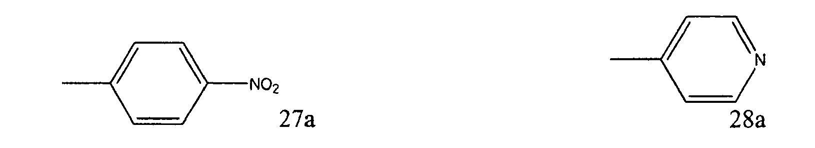 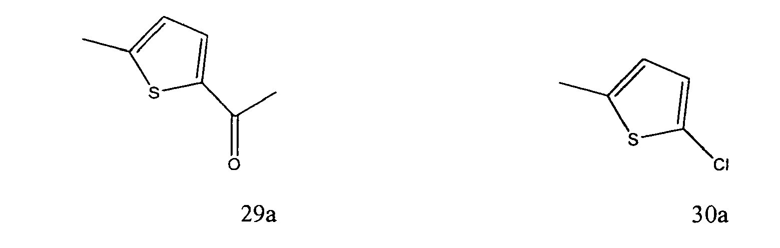 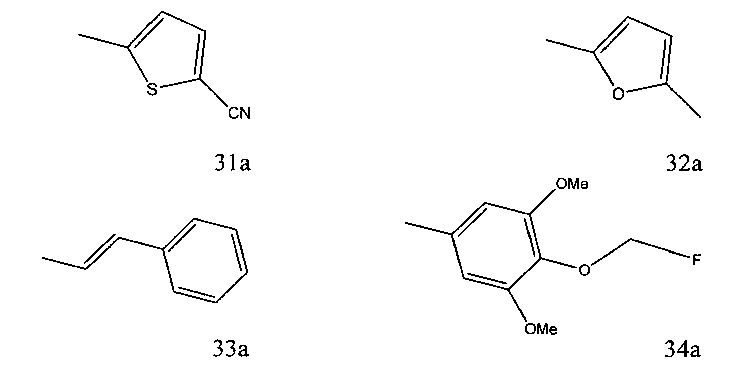 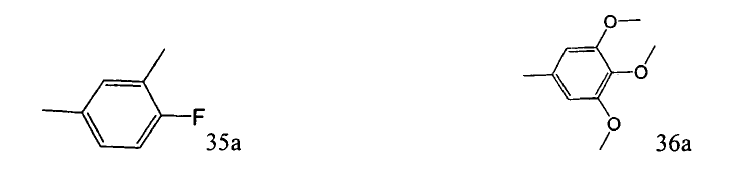 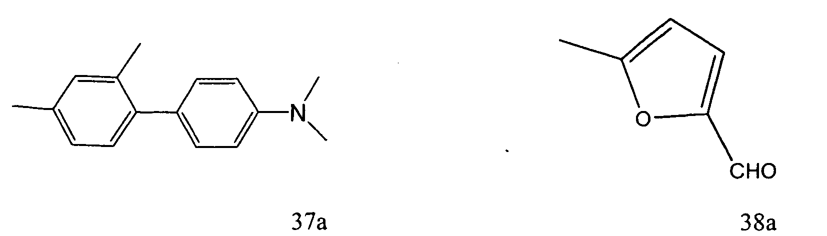 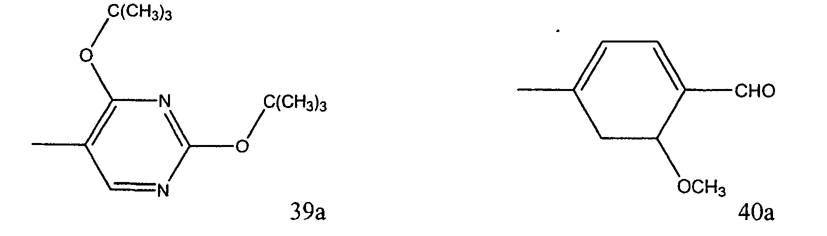 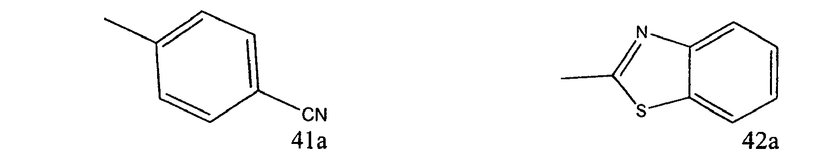 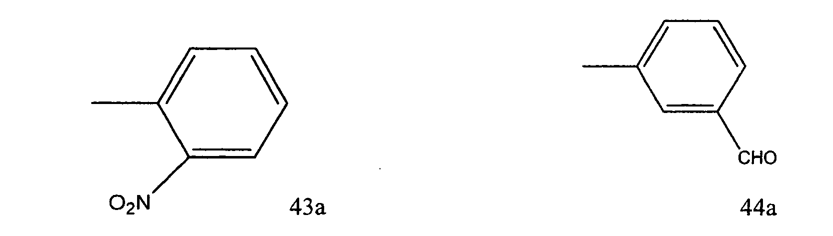 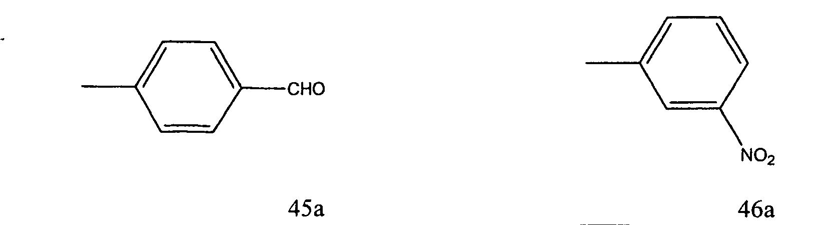 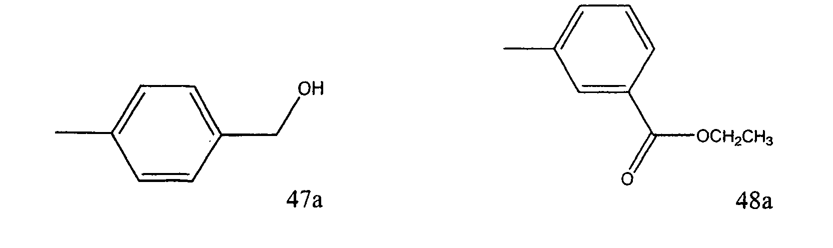 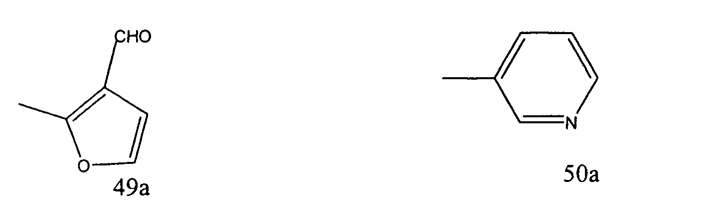 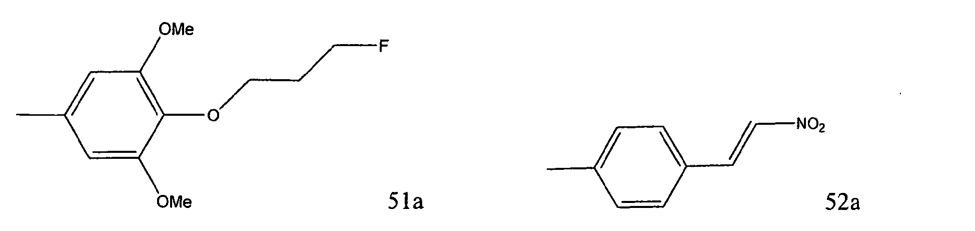 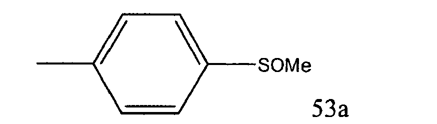 said compound further comprising a label selected from the group consisting of radioisotopes, paramagnetic particles and optical particles.

2. An imaging agent according to claim 1, wherein the label is a radioisotope selected from the group consisting of ³H, ¹¹C, ¹⁴C, ¹⁸F, ³²P, ³⁵S, ¹²³I, ¹²⁵I, ¹³¹I, ⁵¹Cr, ³⁶CI, ⁵⁷Co, ⁵⁹Fe, ⁷⁵Se and ¹⁵²Eu.

3. An imaging agent according to claim 1, wherein the label is a paramagnetic particle selected from the group consisting of ¹⁵⁷Gd, ⁵⁵Mn, ¹⁶²Dy, ⁵²Cr, and ⁵⁶Fe.

4. An imaging agent according to claim 1, wherein the label is an optical particle selected from the group consisting of fluorophores and chemiluminescent entities.

5. A method of detecting at least one of A-beta species and amyloidogenic peptides comprising the steps of
providing a sample suspected of comprising at least one of A-beta species and amyloidogenic peptides;
applying the imaging agent of any one of Claims 1 to 4; and detecting an amount of the imaging agent bound to at least one of A-beta species and amyloidogenic peptides.

6. The method according to claim 5, wherein the A-beta species is soluble A-beta selected from the group consisting of monomers, dimers, trimers, oligomers of up to 24 A-beta peptides, and combinations thereof.

7. A method as in claim 5, wherein the A-beta species is selected from the group consisting of monomers, dimers, trimers, and oligomers of A-beta 1-38, A-beta 1-39, A-beta 1-40, A-beta 1-41, A-beta 1-42, A-beta 1-43 and combinations thereof.

8. The imaging agent of any one of Claims 1 to 4 for use in a method of assessing an amyloid-related disease, wherein said method comprises:
administering to a subject having or suspected of having an amyloid-related disease,
the imaging agent of Claims 1 to 4; and
detecting the imaging agent bound to at least one of A-beta species and amyloidogenic peptides using non-invasive imaging.

9. The imaging agent of claim 8, wherein the soluble A-beta species is as defined in Claim 6 or 7.

10. The imaging agent of claim 8, wherein the amyloid-related disease is Alzheimer's disease.

11. The imaging agent of claim 8, wherein the step of detecting comprises non-invasively measuring the level of the imaging agent within the subject.

12. The imaging agent of claim 8, wherein the step of detecting comprises imaging the brain of the subject.

13. The imaging agent of any one of Claims 1 to 4 for use in the manufacture of a composition for evaluating the effectiveness of a therapy, said method comprising:
administering to a subject a first dose of a composition comprising the imaging agent of Claims 1 to 4;
non-invasively obtaining a baseline measurement of the imaging agent within the subject;
administering to the subject a therapy to be evaluated;
administering to the subject a second dose of said composition;
non-invasively obtaining a second measurement of the imaging agent within the subject; and
comparing the two or more measurements separated in time, wherein an increase or decrease in the amount of the imaging agent present indicates the efficacy of the therapy.

14. The imaging agent of claim 13 wherein the therapy to be evaluated is administered before administration of the first dose of the composition.

15. The imaging agent of claim 13 wherein the first dose of the composition comprises the imaging agent in an amount ranging from 0.1 nmol to about 100 mg.

16. The imaging agent of claim 13, wherein the steps of non-invasively obtaining measurements comprise generating and analyzing an image using a technique selected from the group consisting of positron emission tomography, magnetic resonance imaging, optical imaging, single photon emission computed tomography, ultrasound and x-ray computed tomography.

17. The imaging agent of claim 13, wherein the step of non-invasively obtaining measurements further comprises measuring the amount of imaging agent that is activated by at least one of A-beta species and amyloidogenic peptides.

18. An imaging composition comprising:
the imaging agent of any one of claims 1 to 4; and a pharmaceutically acceptable carrier.

## Patentansprüche

1. Bildgebendes Mittel, welches eine Benzofuranverbindung nach Formel (II) umfasst, 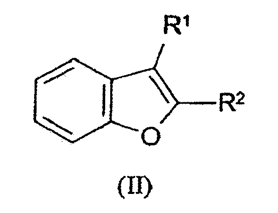 wobei R¹ ein Hydroxyalkyl ist; und R² ein Kohlenwasserstoff-Radikal ist, ausgewählt aus der Gruppe bestehend aus Strukturen 1a bis 53a; 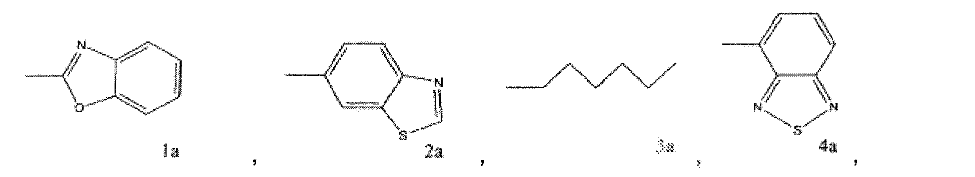 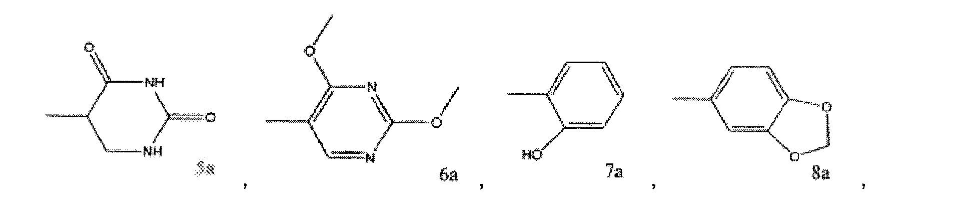 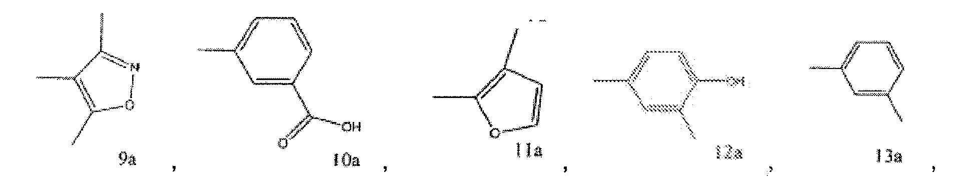 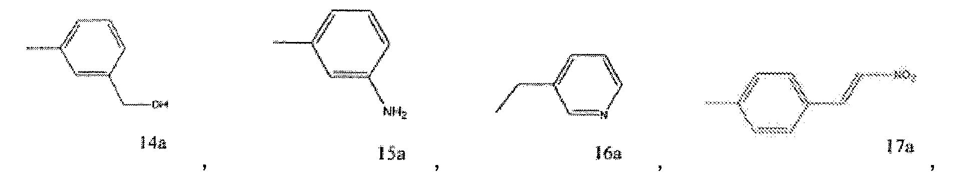 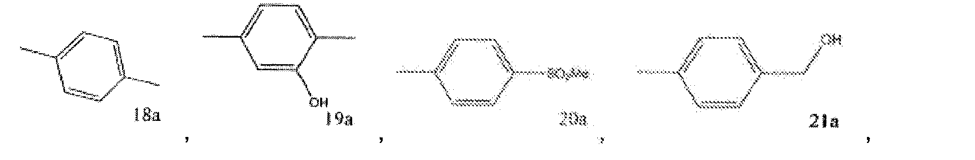 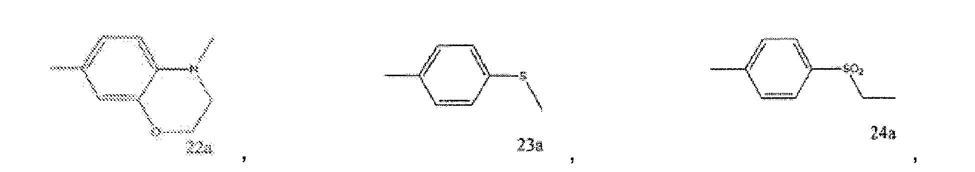 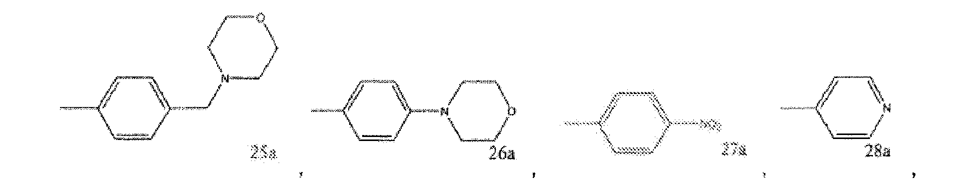 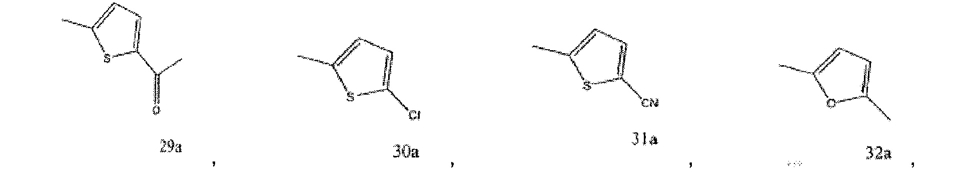 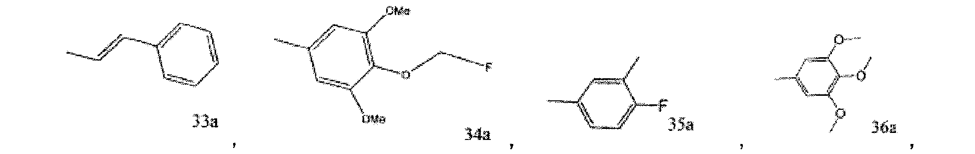 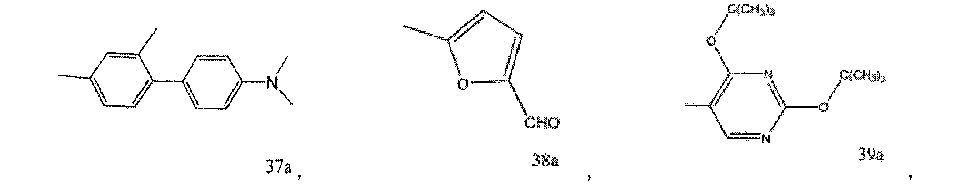 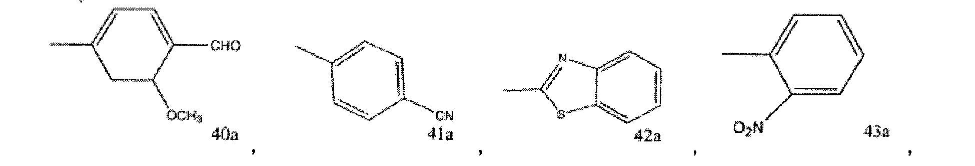 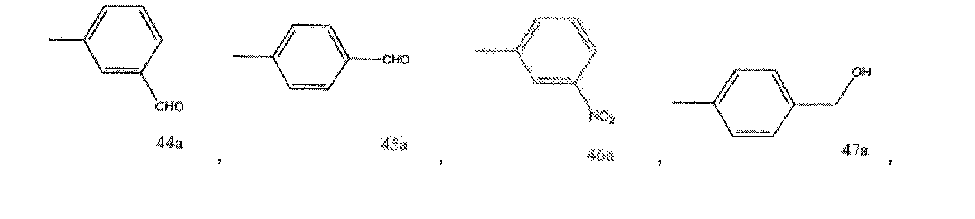 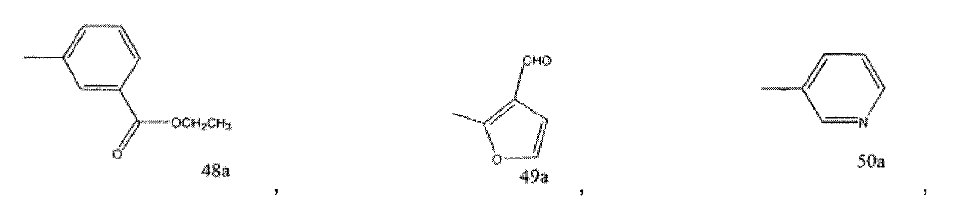 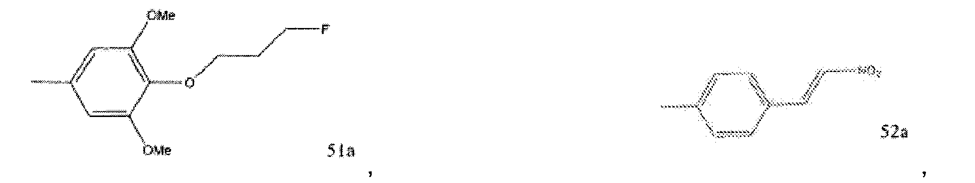 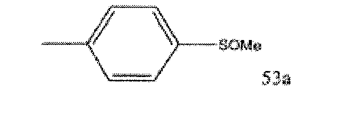 wobei die Verbindung weiterhin eine Markierung umfasst, welche ausgewählt ist aus der Gruppe bestehend aus Radioisotopen, paramagnetischen Partikeln und optischen Partikeln.

2. Bildgebendes Mittel nach Anspruch 1, wobei die Markierung ein Radioisotop ist, ausgewählt aus der Gruppe bestehend aus ³H, ¹¹C, ¹⁴C, ¹⁸F, ³²P, ³⁵S, ¹²³I, ¹²⁵I, ¹³¹I, ⁵³Cr, ³⁶Cl, ⁵⁷Co, ⁵⁹Fe, ⁷⁵Se und ¹⁵²Eu.

3. Bildgebendes Mittel nach Anspruch 1, wobei die Markierung ein paramagnetisches Partikel ist, ausgewählt aus der Gruppe bestehend aus ¹⁵⁷Gd, ⁵⁵Mn, ¹⁶²Dy, ⁵²Cr und ⁵⁶Fe.

4. Bildgebendes Mittel nach Anspruch 1, wobei die Markierung ein optisches Partikel ist, ausgewählt aus der Gruppe bestehend aus Fluorophoren und chemilumineszenten Einheiten.

5. Verfahren zur Detektion wenigstens einer A-beta Spezies und amyloidogenen Peptiden, umfassend die Schritte:
Bereitstellen einer Probe, welche vermutlich wenigstens eine A-beta Spezies und amyloidogene Peptide umfasst;
Anwenden des bildgebenden Mittels nach wenigstens einem der Ansprüche 1 bis 4; und Detektieren der Menge des bildgebenden Mittels, welche an wenigstens eine
der A-beta Spezies und amyloidogenen Peptiden gebunden ist.

6. Verfahren nach Anspruch 5, wobei die A-beta Spezies löslich ist und ausgewählt ist aus der Gruppe bestehend aus Monomeren, Dimeren, Trimeren, Oligomeren von bis zu 24 A-beta Peptiden und Kombinationen daraus.

7. Verfahren nach Anspruch 5, wobei die A-beta Spezies ausgewählt ist aus der Gruppe bestehend aus Monomeren, Dimeren, Trimeren und Oligomeren von A-beta 1-38, A- beta 1-39, A-beta 1-40, A-beta 1-41, A-beta 1-42, A-beta 1-43 und Kombinationen daraus.

8. Bildgebendes Mittel nach einem der Ansprüche 1 bis 4 zur Verwendung in der Herstellung einer Zusammensetzung zur Auswertung der Effektivität einer Therapie einer amyloidbezogenen Krankheit , wobei das Verfahren umfasst:
Verabreichen des bildgebenden Mittels nach den Ansprüchen 1 bis 4 an ein Subjekt, welches eine amyloidbezogene Krankheit aufweist oder vermutlich aufweis; und
Detektieren des bildgebenden Mittels, welches an wenigstens eine der A-beta Spezies und amyloidogenen Peptide gebunden ist, unter Verwendung nichtinvasiver Bildgebung.

9. Bildgebendes Mittel nach Anspruch 8, wobei die lösliche A-beta Spezies wie in Anspruch 6 oder 7 definiert ist

10. Bildgebendes Mittel nach Anspruch 8, wobei die amyloidbezogenen Krankheit die Alzheimer Krankheit ist.

11. Bildgebendes Mittel nach Anspruch 8, wobei der Schritt des Detektierens die nicht-invasive Bestimmung des Spiegels des bildgebenden Mittels in dem Subjekt umfasst.

12. Bildgebendes Mittel nach Anspruch 8, wobei der Schritt des Detektierens das Abbilden des Gehirns des Subjektes umfasst.

13. Bildgebendes Mittel nach einem der Ansprüche 1 bis 4 zur Verwendung in der Herstellung einer Zusammensetzung zur Beurteilung der Effektivität einer
Therapie, wobei das Verfahren umfasst: Verabreichen einer ersten Dosis einer Zusammensetzung umfassend das bildgebende Mittel nach den Ansprüchen 1 bis 4 an ein Subjekt; nicht-invasives Beschaffen einer Basislinienmessung des bildgebenden Mittels im Subjekt; Verabreichen einer zu beurteilenden Therapie an das Subjekt; Verabreichen einer zweiten Dosis der Zusammensetzung an das Subjekt; nicht-invasives Beschaffen einer zweiten Messung des bildgebenden Mittels im Subjekt; und Vergleichen zwei oder mehreren Messungen, welche zeitlich auseinanderliegen, wobei eine Zunahme oder Abnahme in der Menge des vorhandenen bildgebenden Mittels die Effizienz der Therapie anzeigt.

14. Bildgebendes Mittel nach Anspruch 13, wobei die zu bewertende Therapie vor dem Verabreichen der ersten Dosis der Zusammensetzung verabreicht wird.

15. Bildgebendes Mittel nach Anspruch 13, wobei die erste Dosis der Zusammensetzung das bildgebende Mittel in einer Menge von 0,1 mmol bis ungefähr 100 mg umfasst.

16. Bildgebendes Mittel nach Anspruch 13, wobei die Schritte des nicht-invasiven Beschaffens von Messungen das Erzeugen und Analysieren eines Bildes unter Verwendung einer Technik ausgewählt aus der Gruppe bestehend aus Positronenemissionstomographie, magnetischer Resonanzbildgebung, optischem Abbilden, Einzelphotonen-Emission-computerisierte Tomographie, Ultraschall und Röntgenstrahlung-computerisierte Tomographie umfassen.

17. Bildgebendes Mittel nach Anspruch 13, wobei der Schritt des nicht-invasiven Beschaffens von Messungen weiterhin die Bestimmung der Menge des bildgebenden Mittels, welche durch zumindest eine der A-beta Spezies und amyloidogenen Proteinen aktiviert wird, umfasst.

18. Abbildende Zusammensetzung, umfassend: das bildgebendes Mittel nach einem der Ansprüche 1 bis 4; und ein pharmazeutisch geeigneter Träger.

## Revendications

1. Agent d'imagerie qui comprend un composé de coumarone de formule (II) : 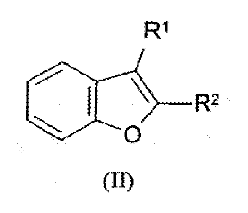 dans laquelle R1 est un hydroxy-alkyle ;et R2 est un radical hydrocarboné sélectionné à partir du groupe constitué par les structures 1a à 53a ; 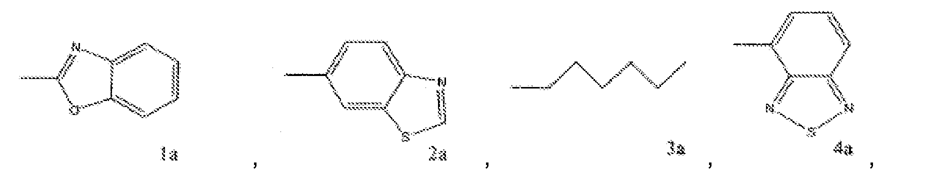 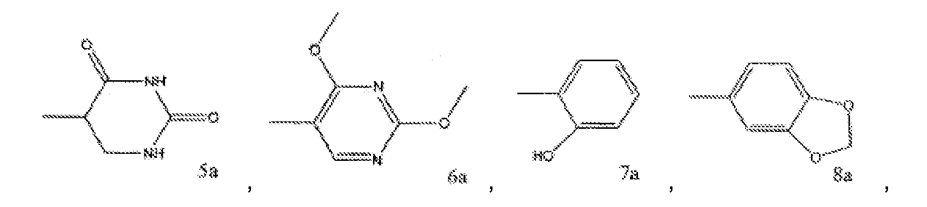 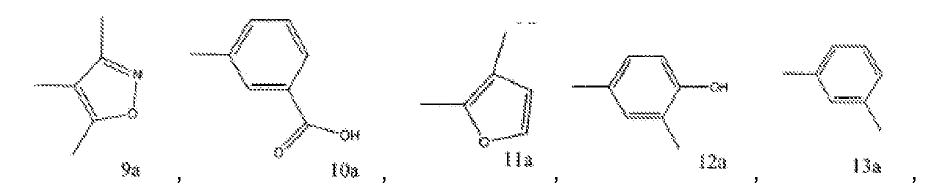 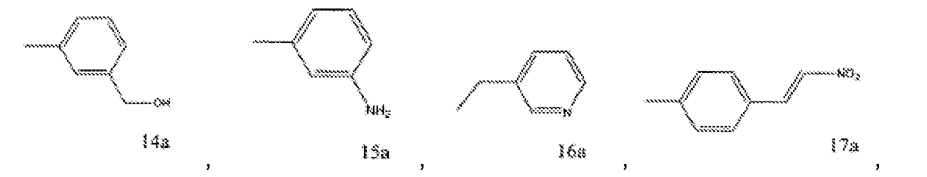 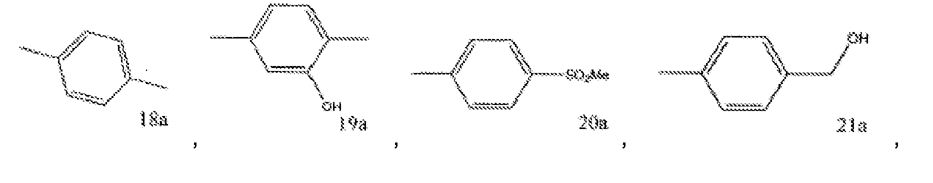 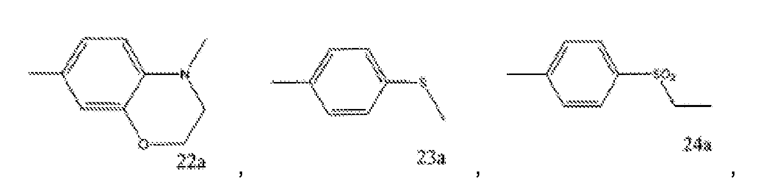 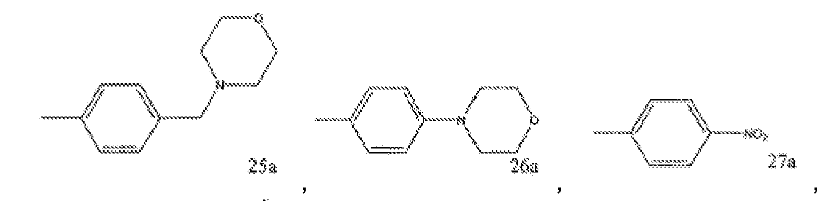 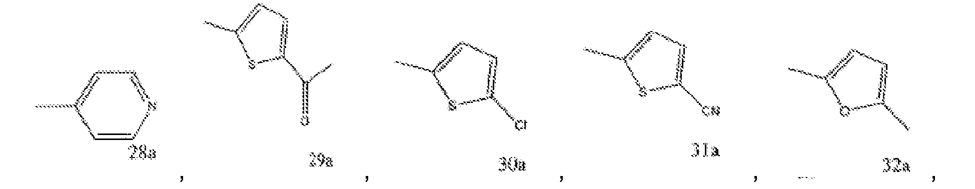 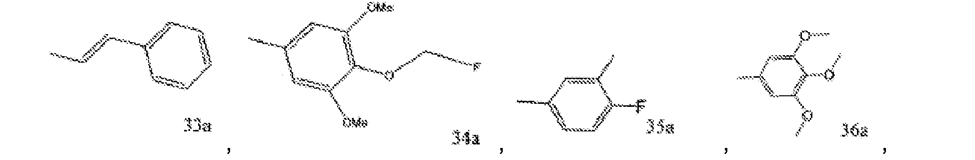 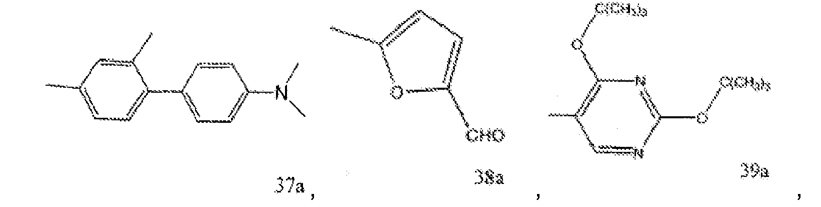 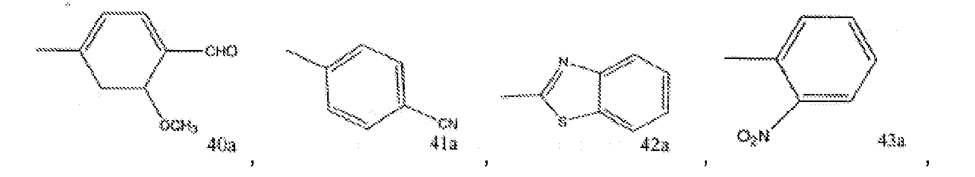 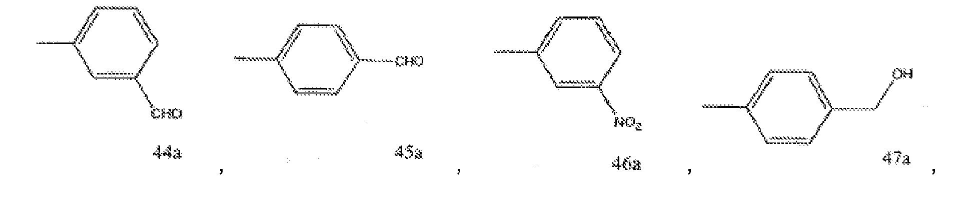 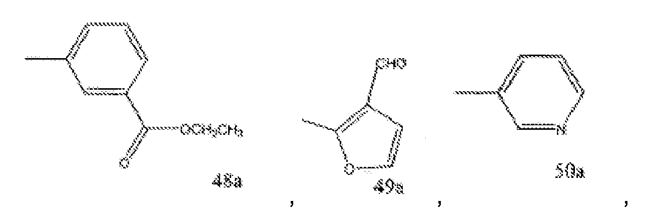 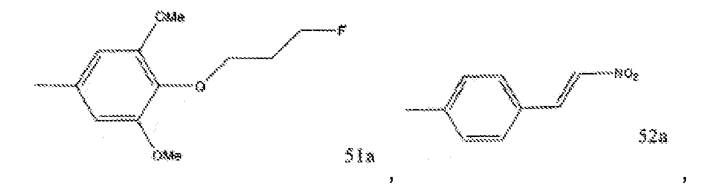 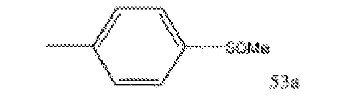 ledit composé comprenant, en outre, un marqueur sélectionné à partir du groupe constitué par des radio-isotopes, des particules paramagnétiques et des particules optiques.

2. Agent d'imagerie selon la revendication 1, dans lequel le marqueur est un radio-isotope sélectionné à partir du groupe constitué par ³H, ¹¹C, ¹⁴C, ¹⁸F_{,} ³²P, ³⁵S, ¹²³I, ¹²⁵I, ¹³¹I, ⁵¹Cr, ³⁶Cl, ⁵⁷Co, ⁵⁹Fe, ⁷⁵Se et ¹⁵²Eu.

3. Agent d'imagerie selon la revendication 1, dans lequel le marqueur est une particule paramagnétique sélectionnée à partir du groupe constitué par ¹⁵⁷Gd, ⁵⁵Mn, ¹⁶²Dy, ⁵²Cr, et ⁵⁶Fe.

4. Agent d'imagerie selon la revendication 1, dans lequel le marqueur est une particule optique sélectionnée à partir du groupe constitué par des fluorophores et des entités chimioluminescentes.

5. Procédé de détection d'au moins l'un d'une espèce d'A-bêta et de peptides amyloïdogènes comprenant les étapes de :
préparation d'un échantillon suspecté de comporter au moins l'un d'une espèce d'A-bêta et de peptides amyloïdogènes ;
application de l'agent d'imagerie selon l'une quelconque des revendications 1 à 4; et
détection d'une quantité de l'agent d'imagerie lié à au moins l'un d'une espèce d'A-bêta et de peptides amyloïdogènes.

6. Procédé selon la revendication 5, dans lequel l'espèce d'A-bêta est soluble, l'A-bêta est sélectionnée à partir du groupe constitué par des monomères, des dimères, des trimères, des oligomères allant jusqu'à 24 peptides A-bêta, et des mélanges de ceux-ci.

7. Procédé selon la revendication 5, dans lequel l'espèce d'A-bêta est sélectionnée à partir du groupe constitué par des monomères, des dimères, des trimères, et des oligomères d'A-bêta 1-38, A-bêta 1-39, A-bêta 1-40, A-bêta 1-41, A-bêta 1-42, A-bêta 1-43 et des mélanges de ceux-ci.

8. Agent d'imagerie selon l'une quelconque des revendications 1 à 4, destiné à une utilisation dans un procédé permettant de déterminer une maladie associée à l'amyloïde, dans lequel ledit procédé comprend :
l'administration à un sujet affecté d'une maladie liée à l'amyloïde ou suspecté de l'être, de l'agent d'imagerie selon les revendications 1 à 4 ; et
la détection de l'agent d'imagerie lié à au moins l'un d'une espèce d'A-bêta et de peptides amyloïdogènes en utilisant un procédé d'imagerie non invasif.

9. Agent d'imagerie selon la revendication 8, dans lequel l'espèce d'A-bêta soluble est selon les revendications 6 ou 7.

10. Agent d'imagerie selon la revendication 8, dans lequel la maladie associée à l'amyloïde est la maladie d'Alzheimer.

11. Agent d'imagerie selon la revendication 8, dans lequel l'étape de détection comprend la mesure non invasive du niveau de l'agent d'imagerie à l'intérieur du sujet.

12. Agent d'imagerie selon la revendication 8, dans lequel l'étape de détection comprend l'imagerie du cerveau du sujet.

13. Agent d'imagerie selon l'une quelconque des revendications 1 à 4 destiné à une utilisation dans la fabrication d'un composé permettant d'assurer l'évaluation de l'efficacité d'une thérapie, ledit procédé comprenant :
l'administration à un sujet d'une première dose d'une composition comprenant l'agent d'imagerie selon les revendications 1 à 4 ;
l'obtention de manière non invasive d'une mesure de référence de l'agent d'imagerie à l'intérieur du sujet ;
l'administration au sujet d'une thérapie à évaluer ;
l'administration au sujet une seconde dose de ladite composition ;
l'obtention, de manière non invasive, d'une seconde mesure de l'agent d'imagerie à l'intérieur du sujet ; et
la comparaison de deux ou plusieurs mesures séparées dans le temps, dans lequel une augmentation ou une diminution de la quantité de l'agent d'imagerie présent indique l'efficacité de la thérapie.

14. Agent d'imagerie selon la revendication 13, dans lequel la thérapie à évaluer est administrée avant l'administration de la première dose de composition.

15. Agent d'imagerie selon la revendication 13, dans lequel la première dose de la composition comprend l'agent d'imagerie en une quantité s'étendant de 0,1 nmol à 100 mg environ.

16. Agent d'imagerie selon la revendication 13, dans lequel les étapes d'obtention de mesures de manière non invasive comprend la production et l'analyser d'une image en utilisant un procédé sélectionné à partir du groupe constitué par la tomographie par émission de positron, l'imagerie par résonance magnétique, l'imagerie optique, la tomographie d'émission monophotonique, la tomographie par rayon X et acoustique.

17. Agent d'imagerie selon la revendication 13, dans lequel l'étape d'obtention de mesures de manière non invasive comprend, en outre, la mesure de la quantité d'agent d'imagerie qui est activée par au moins l'un d'une espèce d'A-bêta et de peptides amyloïdogènes.

18. Composition d'imagerie comprenant :
l'agent d'imagerie selon l'une quelconque des revendications 1 à 4 ; et un support pouvant être accepté pharmaceutiquement.
